# EUROPEAN PATENT SPECIFICATION

(11) **EP 0 802 199 B1**
(45) Date of publication and mention of the grant of the patent: **08.12.1999**
(21) Application number: 97302417.7
(22) Date of filing: 08.04.1997
(51) Int. Cl.: C07K 9/00, A61K 38/14

(54) **Covalently bound dimers of glycopeptide antibiotics**
Kovalentgebundene Dimere von Glycopeptid-Antibiotika
Dimères liés covalemment d'antibiotiques glycopeptidiques

(30) Priority: 12.04.1996 US 15300; 25.11.1996 US 31735
(43) Date of publication of application: 22.10.1997
(73) Proprietor: ELI LILLY AND COMPANY, Indianapolis, Indiana 46285 (US)
(72) Inventor: Stack, Douglas Richard, Fishers, Indiana 46038 (US); Thompson, Richard Craig, 900 West Frankfort, Indiana 46041 (US)
(74) Representative: Hudson, Christopher Mark

(56) References cited:
- JOURNAL OF ANTIBIOTICS, vol. 49, no. 2, February 1996, TOKYO JP, pages 181-193, XP002037248 H LINSDELL ET AL.: "Dimerization of A82846B, vancomxycin and ristocetin, influence on antibniotic complexation with cell wall model pepitdes "
- JOURNAL OF THE AMERICAN CHEMICAL SOCIETY, vol. 115, no. 1, 13 January 1993, DC US, pages 232-237, XP002037249 U GERHARD ET AL. : "The role of the sugar and chlorine substituents in the dimerization of vancomycin antibiotics"
- JOURNAL OF THE AMERICAN CHEMICAL SOCIETY, vol. 118, no. 51, 25 December 1996, DC US, pages 13107-13108, XP002037250 U N SUNDRAM ET AL.: "Novel vancomycin dimers with activity against vancomycin-resistant enterococci"

## Description

The present invention is directed to certain glycopeptide dimers in which two glycopeptide units are covalently linked to one another through their disaccharide amine, via a linking radical. This invention is also directed to the monomeric intermediates. All of these compounds are useful as antibacterials, especially for the control of gram positive bacteria; the compounds are particularly useful for the control of resistant bacterial strains, such as vancomycin-resistant-enterococci ("VRE") .

The compounds of the present invention are defined by the following Formulae I and II: 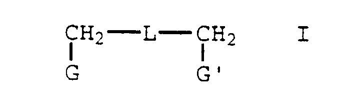 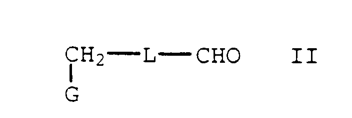 In the above formulae, each of G and G' is independently selected from the group consisting of deshydrovancomycin of the formula: 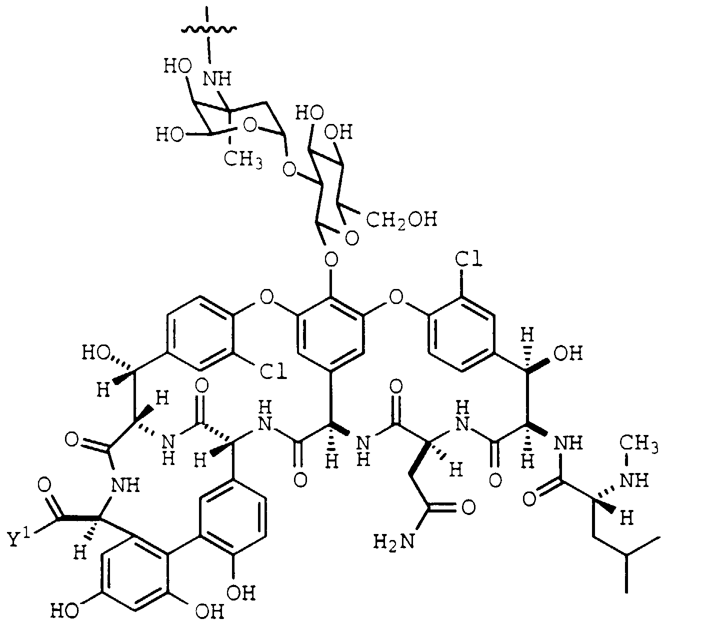 and deshydroA82846B of the formula: 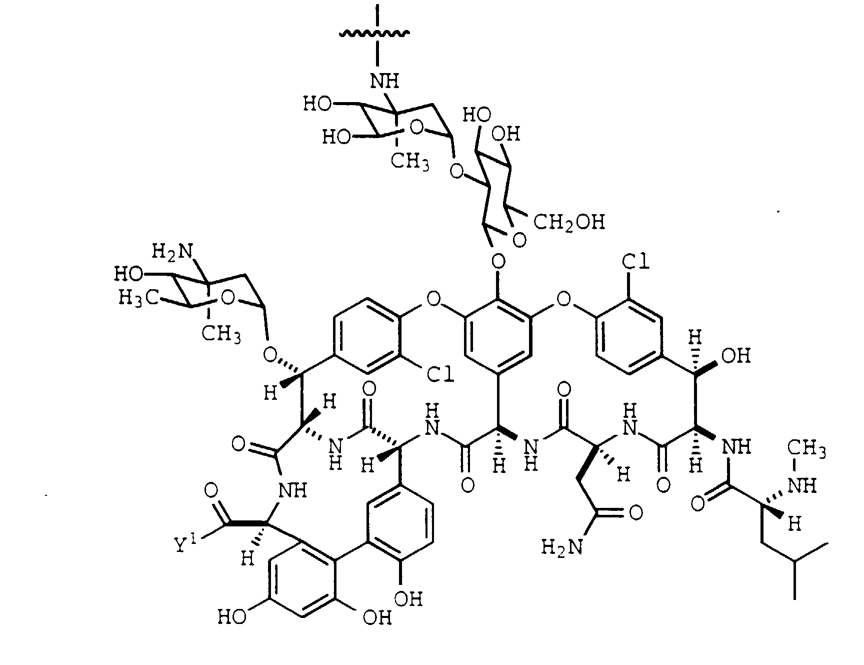 wherein Y¹ is OH or 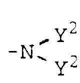 and Y² is defined as follows:
(1) each Y² independently represents
   hydrogen,
   alkyl of C₁-C₁₀,
   cycloalkyl of C₅-C₆.
   cycloalkenyl of C₅-C₆,
   naphthyl,
   biphenylyl,
   radical of the formula -Y³-(Y⁴)_{0, 1, or 2}, wherein Y³ is loweralkyl of C₁-C₆ optionally substituted by from one to three substituents, each of which is independently selected from the group consisting of halo, nitro, cyano, alkoxy, haloalkyl, and haloalkoxy; and Y⁴ is 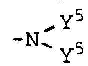 wherein each Y⁵ is independently hydrogen or loweralkyl of C₁-C₄, or Y⁴ is phenyl or phenyl substituted with from one to three substituents, each of which is independently
      halo,
      nitro,
      loweralkyl of C₁-C₄,
      cycloalkyl of C₅-C₆,
      loweralkoxy of C₁-C₄,
      haloloweralkyl of C₁-C₄, or
      haloloweralkoxy of C₁-C₄; or
(2) one Y² is hydrogen and the other Y² is (2-furanon-3-yl); or
(3) both Y²s are taken together with the nitrogen and constitute a five- to seven-membered heterocyclic ring optionally containing in addition to the indicated nitrogen atom one additional hetero ring atom which is nitrogen, oxygen, or sulfur, and which heterocyclic radical can be unsubstituted or substituted with from one or two substituents, each of which is loweralkyl of C₁-C₂, loweralkoxy of C₁-C₂, phenyl, benzyl, or C₁-C₆-alkanoyl; and L is a divalent linking radical of the formula A: 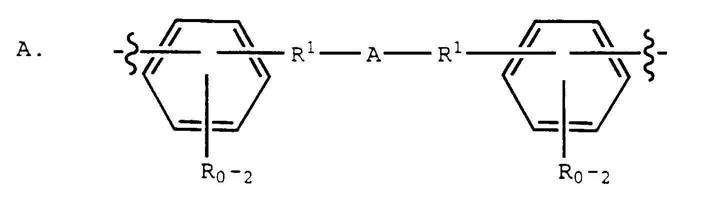
   wherein A is:
      alkylene of C₁-C₁₆,
      (alkylene of C₁-C₄-X')_{q}-alkylene of C₁-C₄, wherein q is 1-3,
      alkylene of 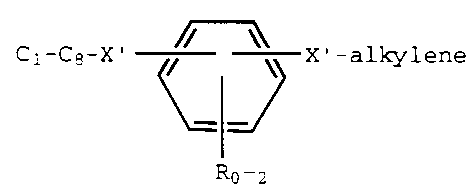 of C₁-C₈;
      alkylene of 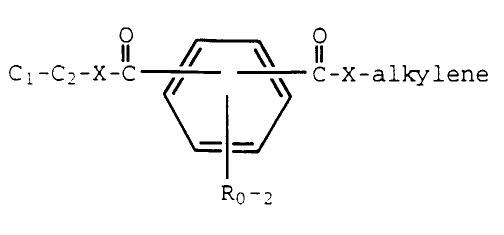 of C₁-C₂, or
      alkylene of 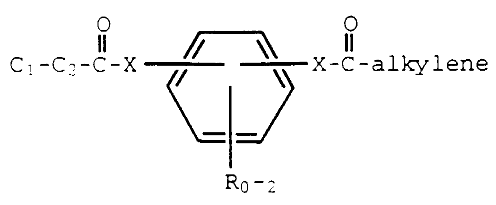 of C₁-C₂ ;
      each R¹ is independently
      CH_{2,}
      O,
      S, 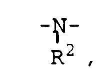 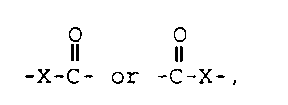 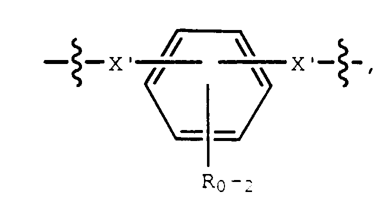 or 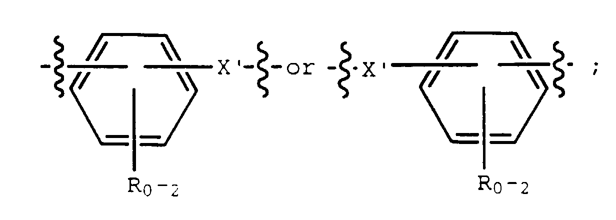
      wherein each R independently represents halo, loweralkyl of C₁-C₆, loweralkoxy of C₁-C₆, phenyl, or phenyl substituted by from 1 to 2 substituents, each of which is independently halo, loweralkyl of C₁-C₆, or loweralkoxy of C₁-C₆; each X is independently -O- or 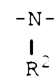 wherein R² is H or loweralkyl of C₁-C₄; and each X' is independently -O-, -S-, or 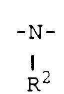 wherein R² is as defined above; or L is a divalent linking radical of the formula B:
B. -alkylene of C₁-C₈-R³-X"-R³-alkylene of C₁-C₈-wherein X" represents alkylene of C₁-C₄ or a phenylene of the formula 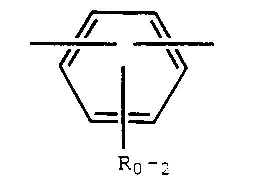 wherein R is as defined above; and each R³ is independently CH₂ or O.

The present invention also includes salts of the foregoing compounds.

In compounds of Formula I, the glycopeptide units, G and G', may be identical or different. In compounds of both Formulae, linkage of the glycopeptide units is through the amine group of the disaccharide sugar. Any "alkylene" of C₂ or higher can be straight chain or branched.

Certain compounds of the present invention are preferred. Compounds of Formula I, and especially symmetrical compounds (G=G' and/or both R¹ are identical), are preferred for their more efficient synthesis.

Antibacterial activity is enhanced by employing preferred "L" groups. Preferences include the following, individually and in any combination:
L = a linking radical of formula A
L = a linking radical of formula B wherein the carbon attached to -CH₂-G or to -CH₂-G' is branched
R¹ = O
A = alkylene of C₁-C₁₆, especially straight-chain and especially C₆-C₁₂;
A = (alkylene of C₁-C₄-X')_{q}-alkylene of C₁-C₄, especially wherein X'=O; the alkylene is -(CH₂)₂-; and q=2;
R = phenyl and substituted phenyl, especially chlorophenyl; and especially when R has this value on a phenyl ring within "A".
Other preferences will be apparent from the further teachings herein.

Representative compounds of the present invention are set forth in following TABLES 1 and 2. TABLE 1 identifies dimers of Formula I; TABLE 2 identifies compounds of Formula II.

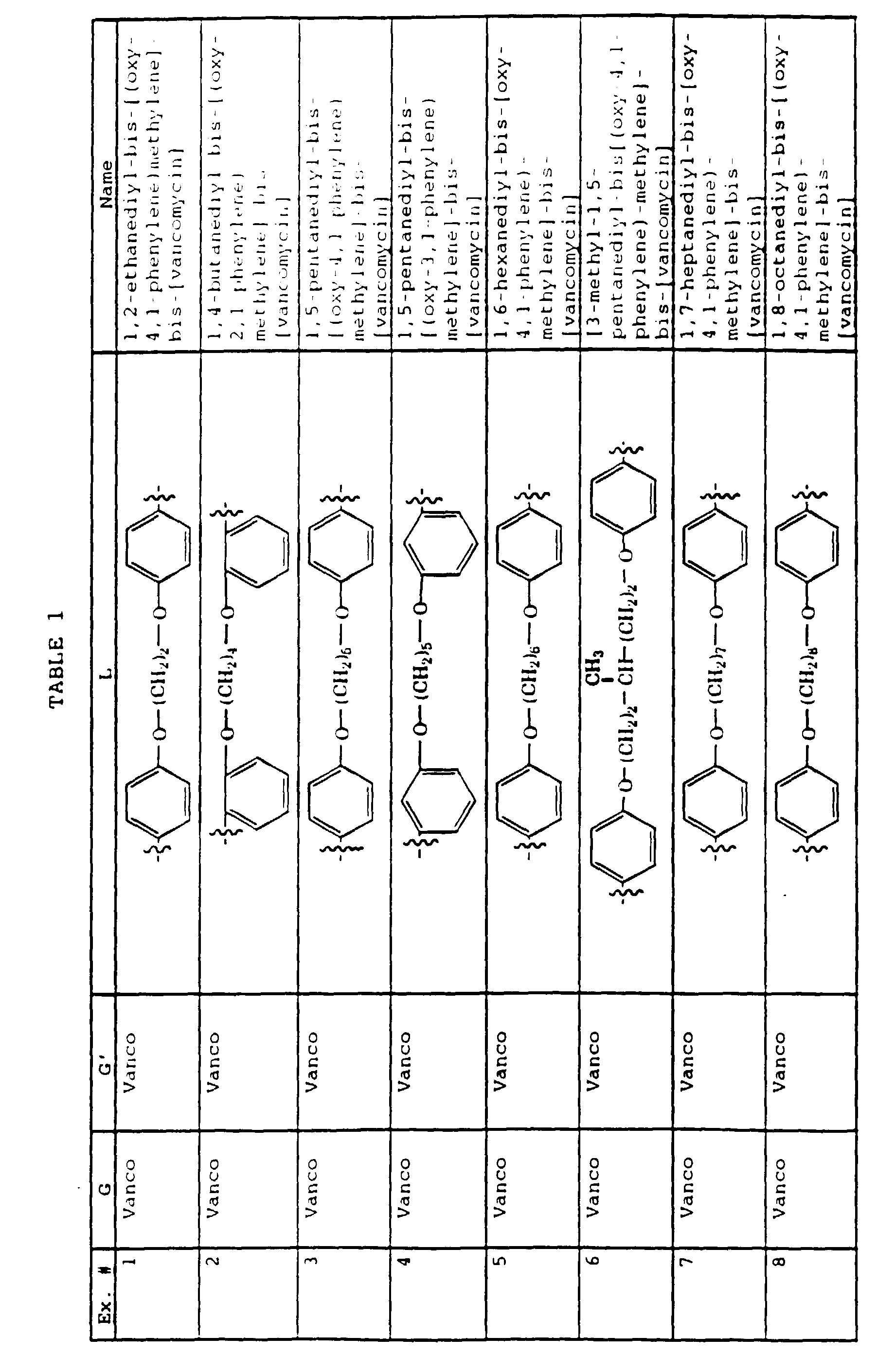

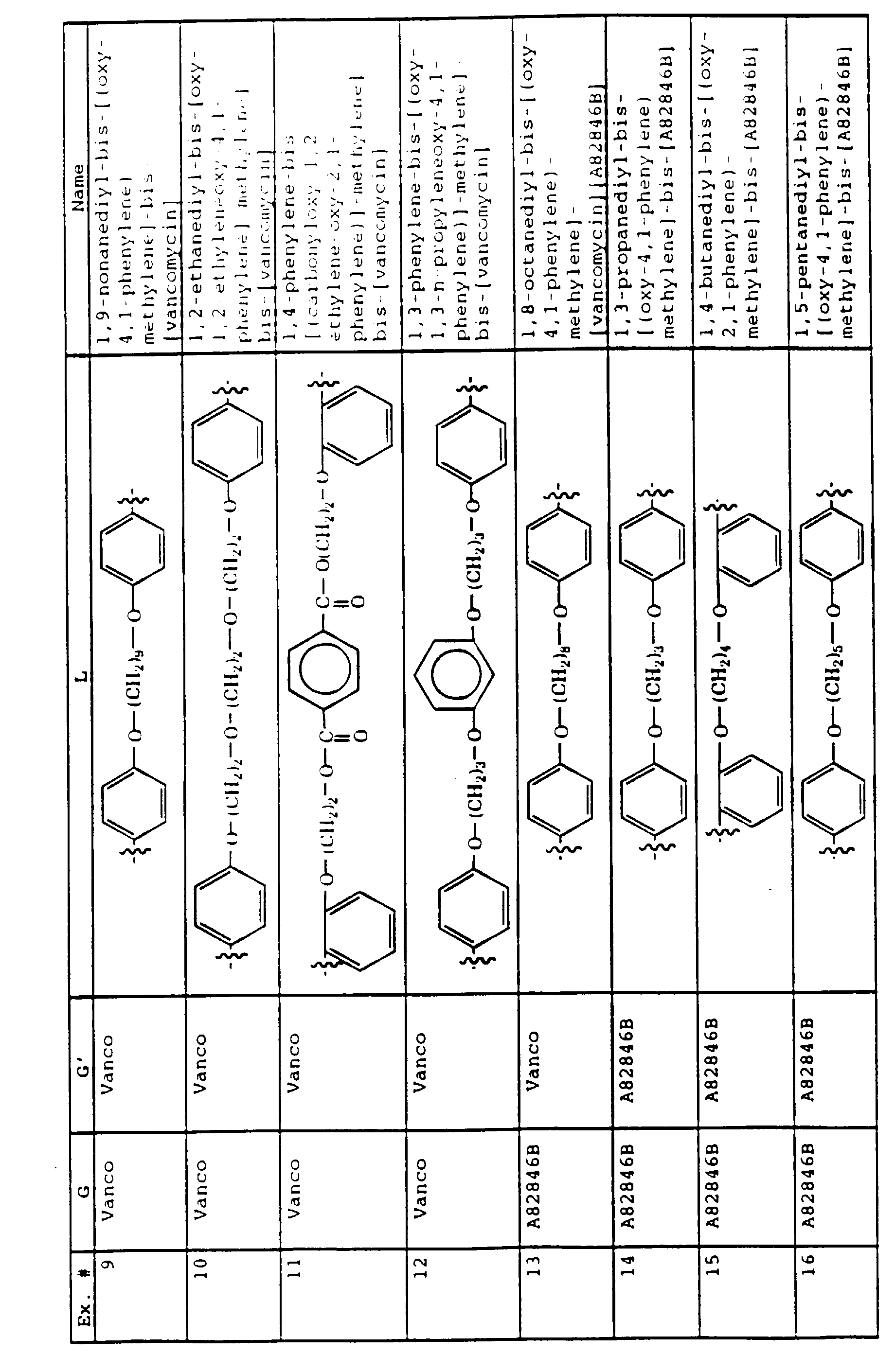

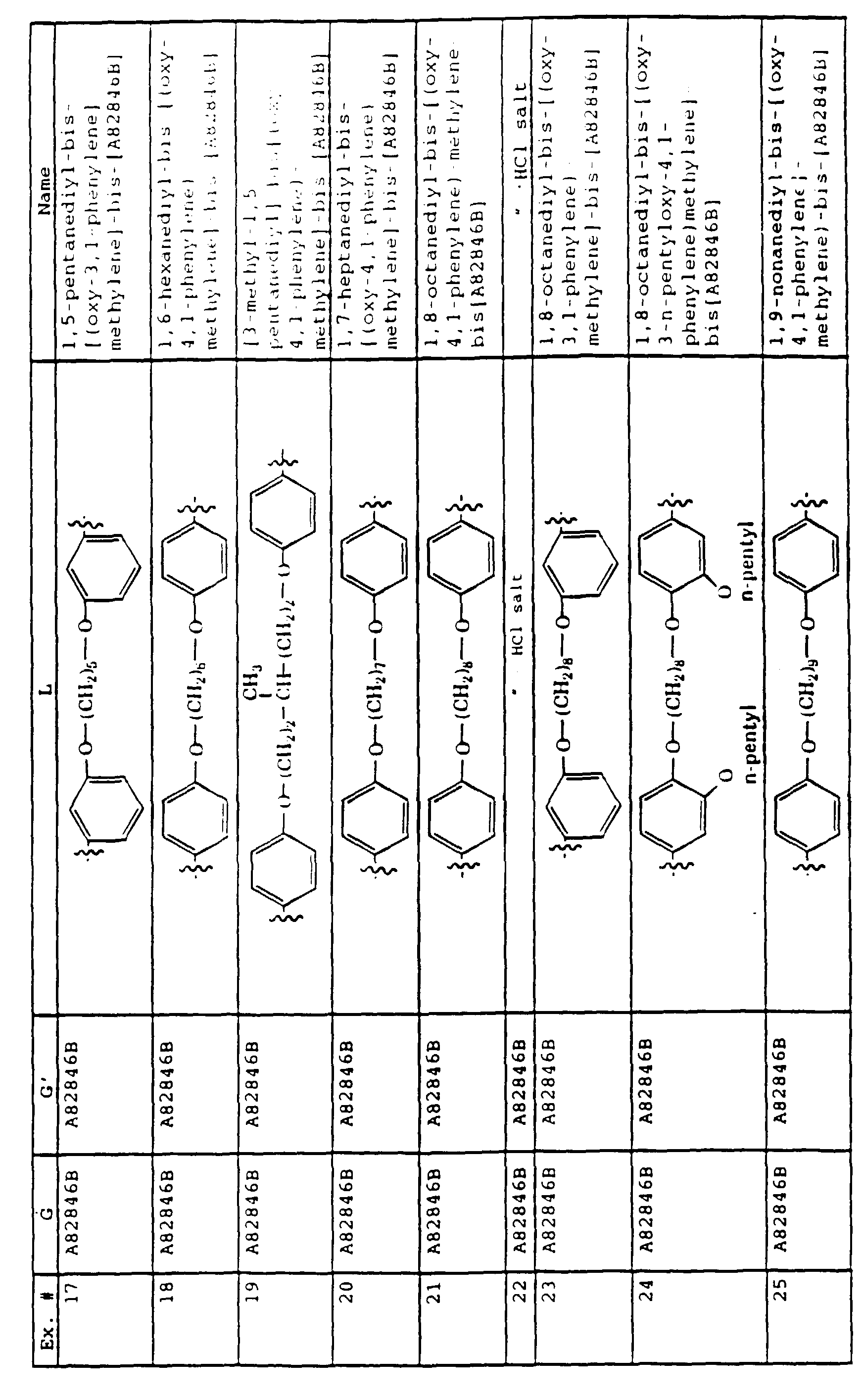

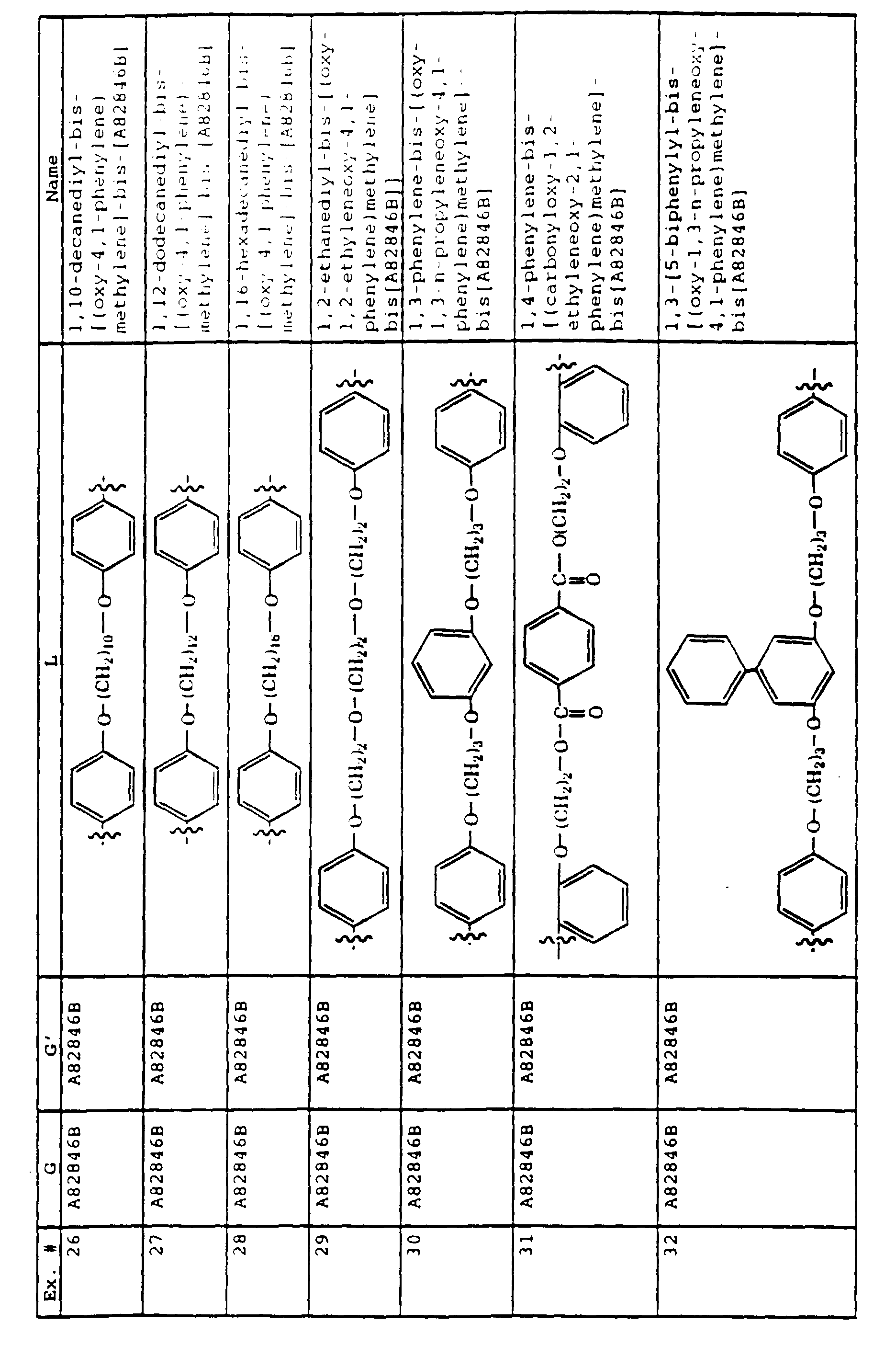

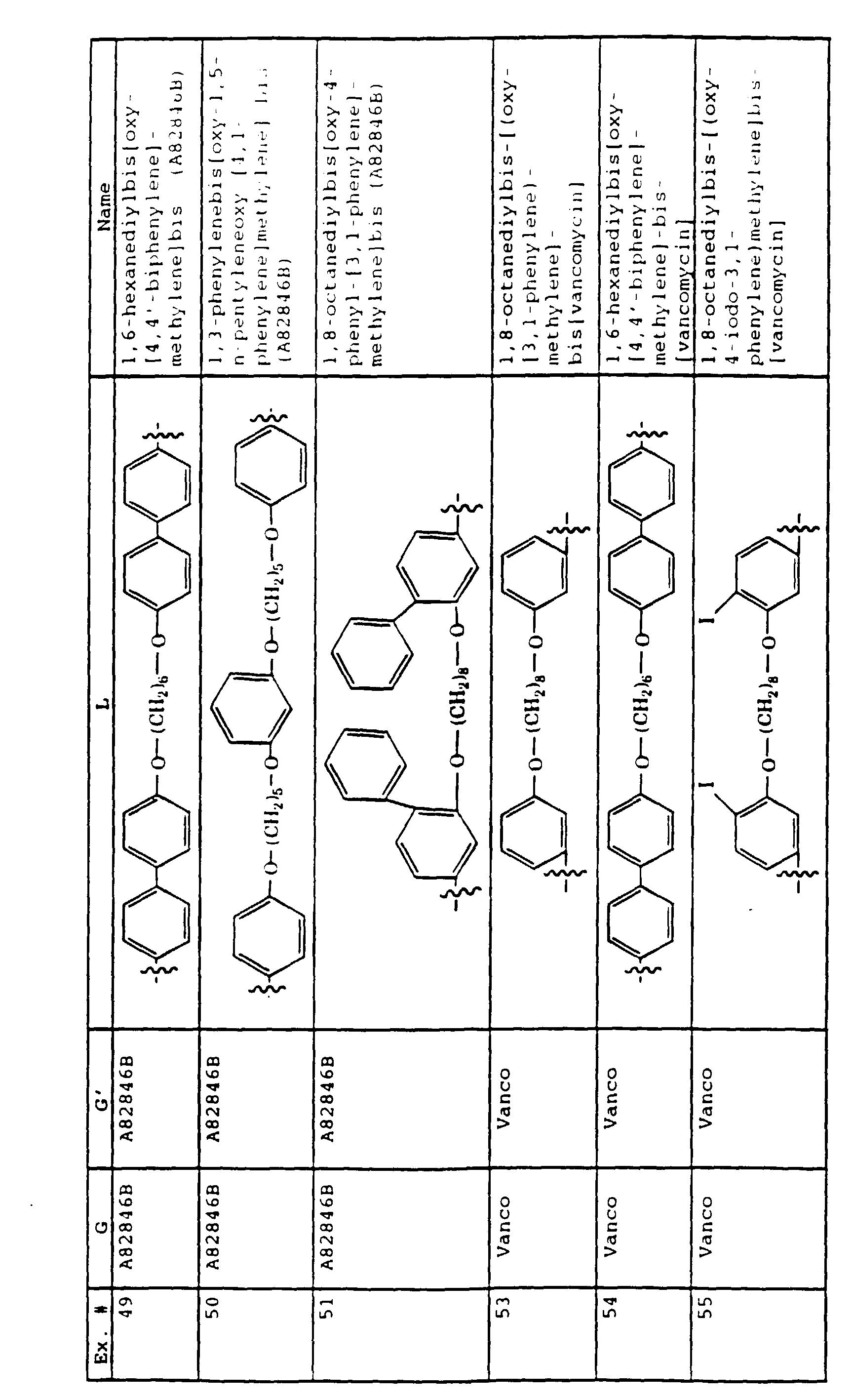

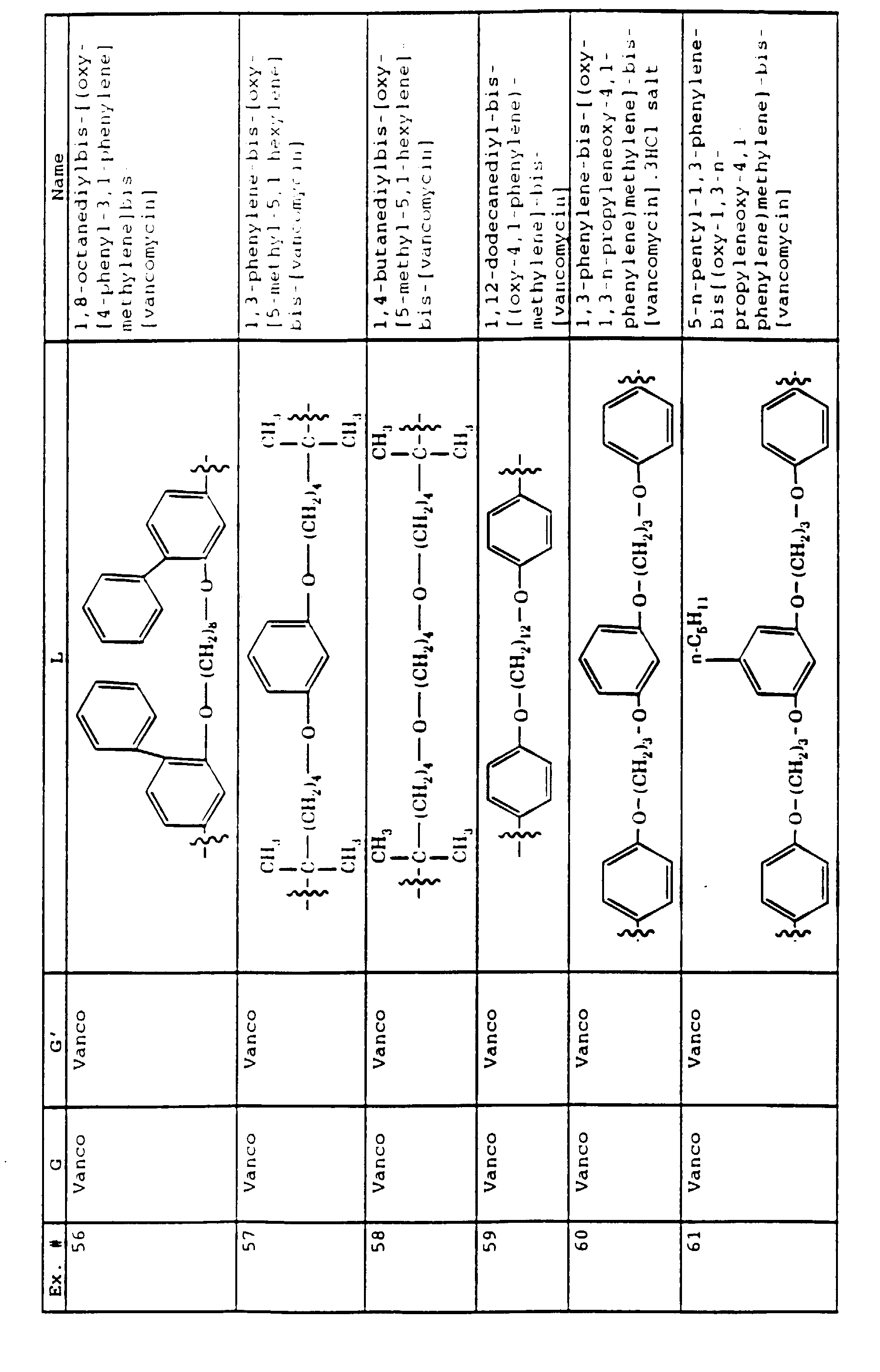

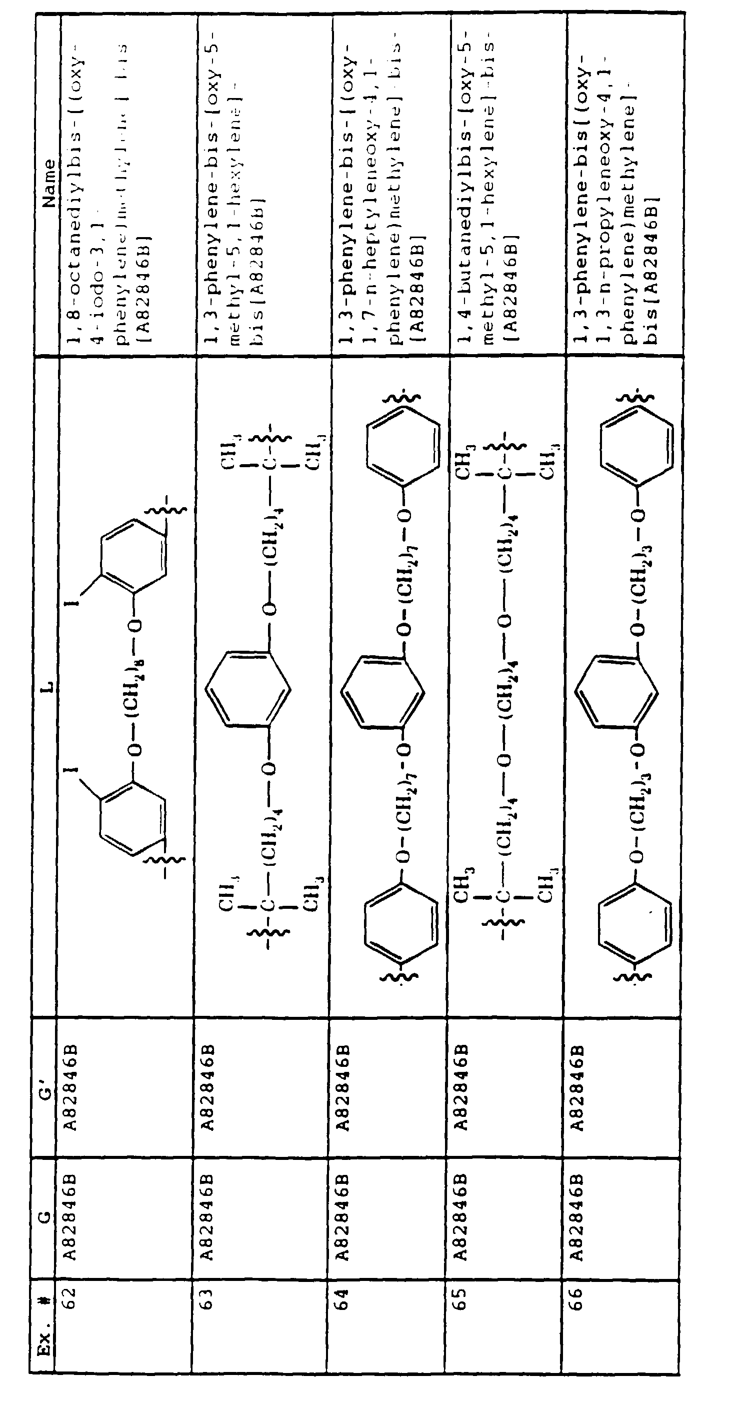

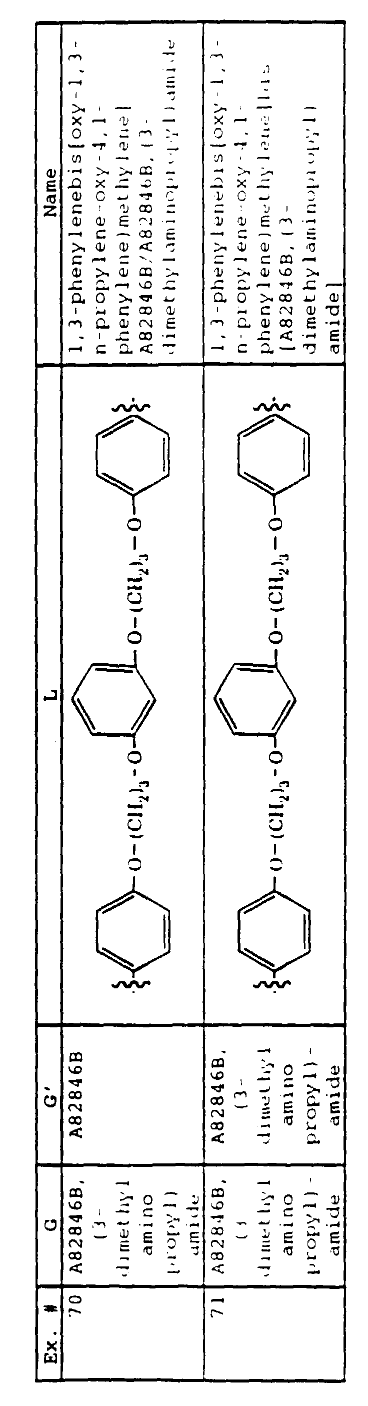

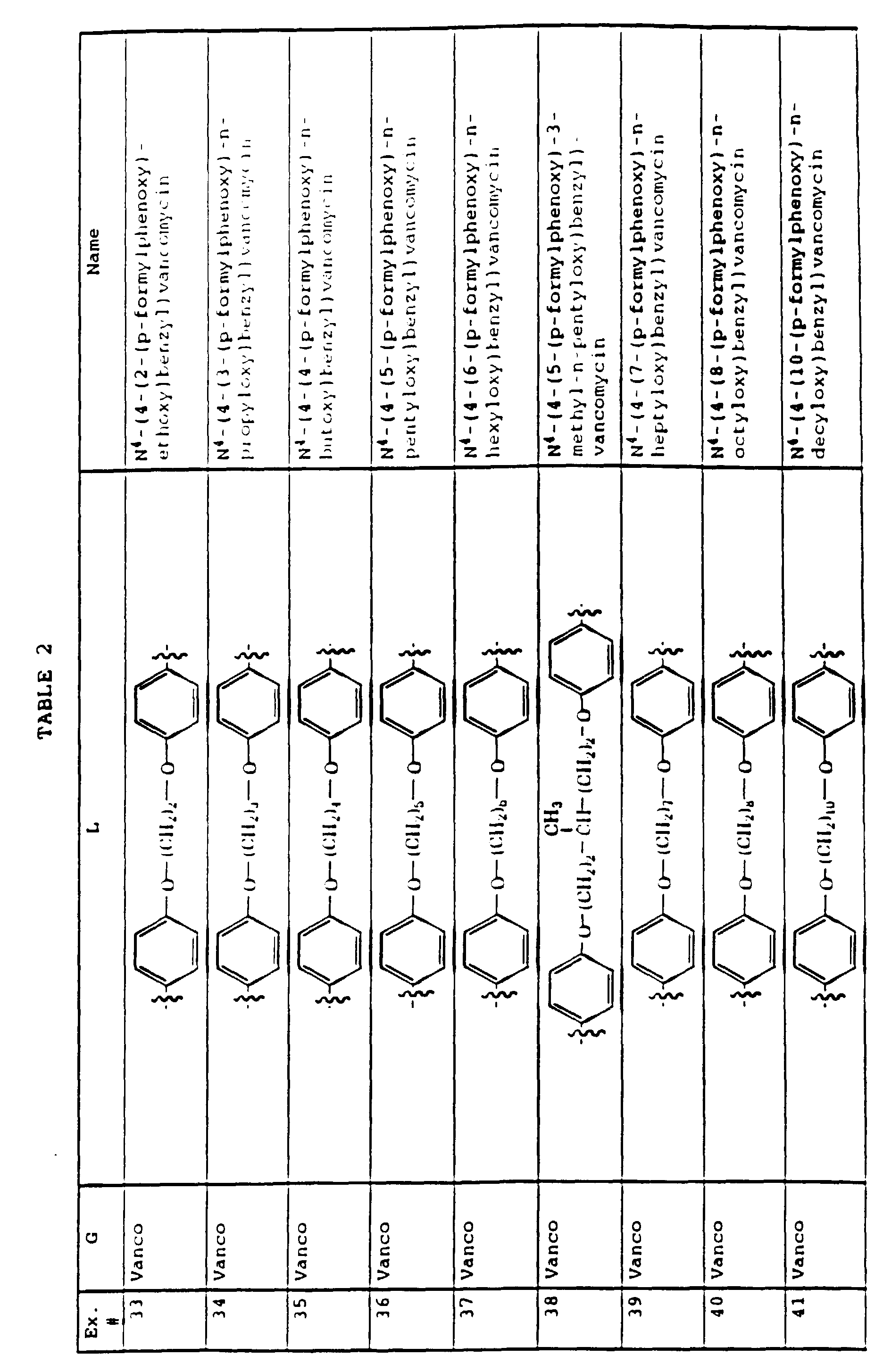

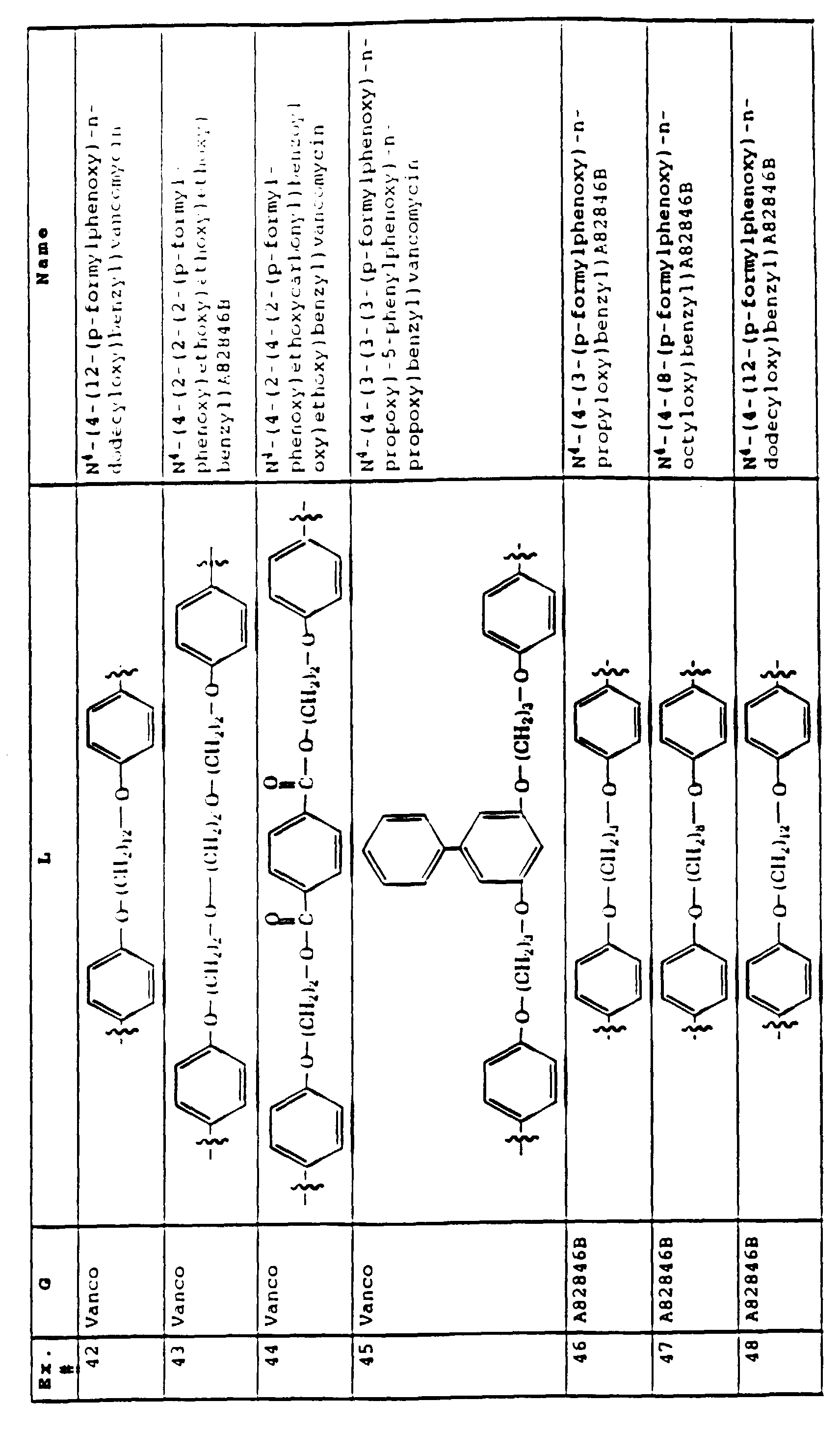

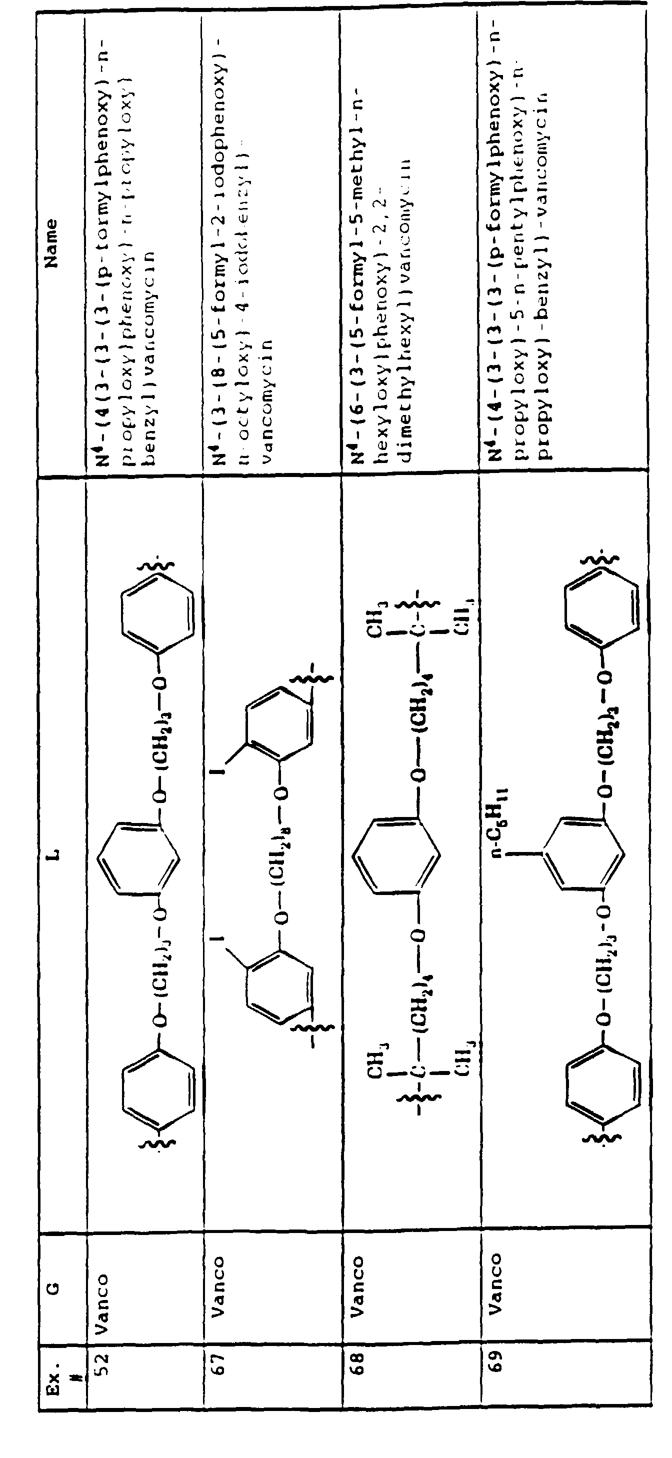

The compounds of the present invention are prepared by reacting vancomycin or A82846B with a bisaldehyde of the formula: 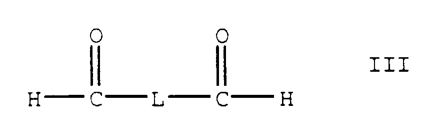 to form an intermediate Schiff base, which is subsequently reduced to obtain the compounds of Formula I and II.

Many of the bisaldehydes to be employed as starting materials are known compounds. All of them can be prepared by techniques known to those skilled in the art, per various references;
J. Org. Chem., **26**, 474, (1961)
J. Het. Chem., **27**, 1007 (1990)
J.A.C.S., **73**, 1872 (1951)
J.A.C.S., **109**, 2260 (1987)
J. Chem. Soc. Perkin I, 189 (1983)
Syn. Comm., **18(12),** 1379 (1988)
Chem. Letters, 587 (1995)
Macromolecules, 6045 (1992);
J. Chem. Soc. Chem. Comm. 1463 (1991)
J. Polym. Sci. Part A, Polymer Chem. 31(12) 2899 (1993)
J. Chem. Res. Synop. (8), 296 (1994)
Farmco Ed. Sci. 15, 468 (1960)
Makromol. Chem. 191 (4) 815 (1990)
J. Polym. Sci. Part A, Polymer Chem. 29(3) 361 (1991)
Makromol. Chem. 65, 54 (1963)

The reaction of bisaldehyde with vancomycin or A82846B is carried out in accordance with prior art condensations of amine and aldehyde to form Schiff bases, and their subsequent reduction.

Thus, the present condensation is typically conducted in a polar solvent, such as dimethylformamide or methanol, or a mixture of polar solvents. The reaction goes forward over a range of temperatures, such as from 25°C to 100°C, but is preferably conducted at temperatures of about 60°C to 70°C. The reaction is preferably conducted under an inert atmosphere, such as nitrogen or argon.

The reaction yields a Schiff base of the formula

G=CH-L-CH=G'

where both aldehyde groups have reacted with glycopeptide, or of the formula

G=CH-L-CHO

where only one aldehyde group has reacted with glycopeptide.

The Schiff base is subsequently reduced. Preferably, the reduction is conducted in the same reaction mixture in a polar solvent, and employing a chemical reducing agent. Metal borohydrides, such as sodium borohydride and sodium cyanoborohydride are preferred. The reaction goes forward over a range of temperatures, such as from about 25°C to about 100°C; preferably, the reaction is conducted at about 60°C to 70°C.

Depending somewhat on concentration of reagents, the condensation of bisaldehyde with vancomycin or A82846B and subsequent reduction will yield a dimer of Formula I, a monosubstituted derivative of Formula II, or a mixture of both. Generally, both products are produced. However, some control of the products can be achieved by the amount of reactants employed. A dimer of Formula I requires two molecular proportions of vancomycin or A82846B per molecular proportion of bisaldehyde, whereas a compound of Formula II requires equimolar amounts of the reactants. Preferably the reaction is continued through the reduction, and the respective products separated at that time.

The product, or mixture of products, can be isolated and purified if desired in a conventional manner, such as by HPLC. Characterization of products is best accomplished by Fast Atom Bombardment Mass Spectroscopy (FAB•MS).

In addition to the foregoing synthetic route, compounds of the present invention can be prepared in an alternate route. In this alternate route, a dimer is prepared by the foregoing synthetic route, and further changes to the structure of the glycopeptide are made subsequently. This approach to synthesizing the present dimers is illustrated by Preparations 7 and 8 below in which a dimer of the present invention is reacted with an amine to convert the acid of the glycopeptide to an amide 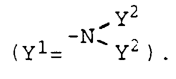 Other modifications of the glycopeptide portion of a dimer can likewise be made. Techniques for such modifications are known to those skilled in the art; see Glycopeptide Antibiotics, edited by Ramakrishnan Nagarajan (Marcel Dekker, Inc., New York, 1994), and references cited therein. This volume is incorporated herein by reference.

When it is desired to employ a salt, a compound of the present invention can be reacted with a mineral or organic acid or an inorganic base, in techniques well known to those skilled in the art. Pharmaceutically-acceptable salts are preferred.

The following examples report preparations of illustrative compounds of the present invention.

The HPLC procedures reported in these examples were as follows:

Analytical ("Conditions A"): Reactions were monitored by analytical HPLC using a Waters µBondapak C₁₈ column (3.9x300 mm) and UV detection at 280 nm. Elution was accomplished with a linear gradient of 5% CH₃CN - 95% buffer to 80% CH₃CN - 20% buffer over 30 minutes. The buffer used was 0.5% triethylamine in water, adjusted to pH 3 with H₃PO₄.

Preparative ("Conditions B"): Crude reaction mixtures were purified by preparative HPLC using a Waters C₁₈ Nova-Pak column (40x300 mm) and UV detection at 280 nm. Elution was accomplished with a linear gradient of 5% CH₃CN - 95% buffer to 80% CH₃CN - 20% buffer over 30 minutes. The buffer used was 0.5% triethylamine in water, adjusted to pH 3 with H₃PO₄. The desired fractions were subsequently desalted with a Waters C₁₈ Sep-Pak (35 cc) followed by lyophilization. Alternatively, a buffer containing 0.1% TFA in H₂O can be used, in which case the TFA salt is obtained directly after lyophilization.

Compounds were desalted as follows. A Waters Sep-Pak cartridge was pre-wet with methanol (2-3 column volumes) then conditioned with water (2-3 column volumes). The sample, dissolved in a minimum volume of water, was loaded onto the Sep-Pak column which was then washed with water (2-3 column volumes) to remove the unwanted salts. The product was then eluted with an appropriate solvent system, typically 1:1 CH₃CN/H₂O, CH₃CN, and/or methanol. The organic solvent component was removed *in vacuo* and the resulting aqueous solution lyophilized to give the final product.

### Preparation 1:

### Synthesis of Example 5, 1,6-hexanediylbis[(oxy-4,1-phenylene)methylene]bis[vancomycin]

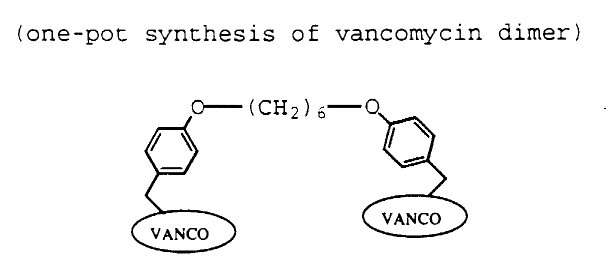

A dry 100 mL round bottom flask was charged with vancomycin•HCl (250 mg, 0.168 mmol.), and 1,6-bis(4'-formylphenoxy)-n-hexane (101 mg, 0.310 mmol.). Anhydrous DMF (6 mL) was added to the flask and the resulting mixture was stirred under N₂ and heated to 70°C. After 3.5 hours, sodium cyanoborohydride (80 mg, 1.3 mmol.) was added in one portion, and the reaction mixture was maintained at 70°C for one additional hour. The reaction mixture was cooled, and stored at 0°C overnight.

The reaction mixture was then concentrated in vacuo to give a residue which was re-dissolved in 1:1 H₂O:CH₃CN (5 mL) and HOAc (0.5 mL). The resulting solution was purified by preparatory HPLC (conditions B). The desired fractions, as determined by analytical HPLC (conditions A), were concentrated in vacuo to - 1.5 mL, and desalted. After lyophilization, 1,6-hexanediylbis[(oxy-4,1-phenylene)methylene]bis[vancomycin] was obtained (24.3 mg, 0.008 mmol., 10.0 % yield) as a white powder.

HPLC (conditions A) retention time: 13.6 min.

FABMS shows peak of (M+6H) at 3195.

### Preparation 2:

### Synthesis of Example 25, 1,9-nonanediylbis[(oxy-4,1-phenylene)methylene]bis[A82846B]

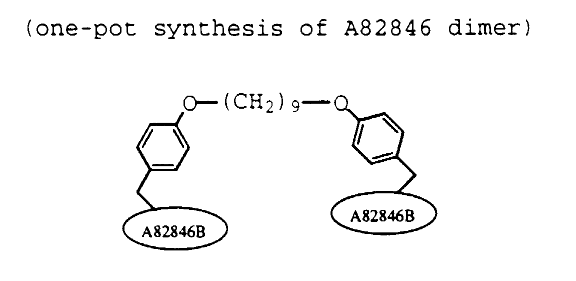

A dry 100 mL round bottom flask was charged with A82846B·tri-acetate salt (278 mg, 0.157 mmol.), and 1,9-bis-(4'-formylphenoxy)-n-nonane (103.7 mg, 0.282 mmol.). Anhydrous DMF (15 mL) and anhydrous MeOH (15 mL) were added to the flask and the resulting mixture was stirred under N₂ and heated to 70°C. After 3.5 hours, sodium cyanoborohydride (68 mg, 1.08 mmol.) was added in one portion, and the reaction mixture was maintained at 70°C for one additional hour.

The reaction mixture was then concentrated in vacuo to give a residue which was re-dissolved in 1:1 H₂O:CH₃CN (5 mL) and HOAc (0.5 mL). The resulting solution was purified by preparatory HPLC (conditions B). The desired fractions, as determined by analytical HPLC (conditions A), were concentrated in vacuo to ∼ 1.5 mL, and desalted. After lyophilization, 1,9-nonanediylbis[(oxy-4,1-phenylene)methylene]bis[epivancomycin] was obtained (25.7 mg, 0.007 mmol., 9.3 % yield) as a white powder.

HPLC (conditions A) retention time: 14.9 min.

FABMS shows peak of (M+5H)at 3522.

### Preparation 3:

### Synthesis of Example 47, N⁴-(4-(8-(p-formylphenoxy)-n-octyloxy)benzyl)A82846B

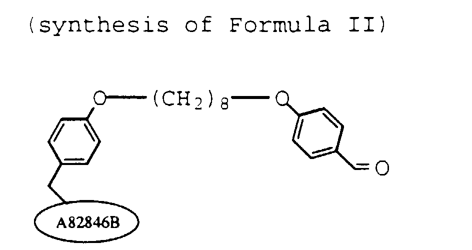

A dry 100 mL round bottom flask was charged with A82846B·tri-acetate salt (278 mg, 0.157 mmol.), and 1,8-bis-(4'-formylphenoxy)-n-octane (100 mg, 0.19mmol.). Anhydrous DMF (15 mL) and anhydrous MeOH (15 mL) were added to the flask and the resulting mixture was stirred under N₂ and heated to 70 °C. After 3.5 hours, sodium cyanoborohydride (48 mg, 0.739mmol.) was added in one portion, and the reaction mixture was maintained at 70 °C for one additional hour.

The reaction mixture was then concentrated in vacuo to give a residue which was re-dissolved in 1:1 H₂O:CH₃CN (5 mL) and HOAc (0.5 mL). The resulting solution was purified by preparatory HPLC (conditions B). The desired fractions, as determined by analytical HPLC (conditions A), were concentrated in vacuo to ∼ 1.5 mL, and desalted. After lyophilization, N⁴-(4-(8-(p-formylphenoxy)-n-octyloxy)benzyl)A82846B was obtained (26.3mg, 0.013 mmol., 8.6% yield) as a white powder.

HPLC (conditions A) retention time:

FABMS shows peak of (M+ 2H) at 1930.

### Preparation 4:

### Synthesis of Example 13, 1,8-octanediylbis[(oxy-4,1-phenylene)methylene][vancomycin][A82846B]

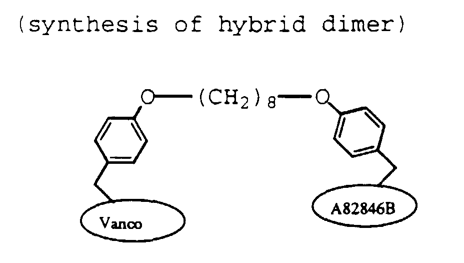

A dry round bottom flask was charged with vancomycin•HCl (75 mg, 0.052 mmol.), and N⁴-(4-(8-(p-formylphenoxy)-n-octyloxy)benzyl)A82846B (50 mg, 0.026 mmol.). Anhydrous DMF (6 mL) was added to the flask and the resulting mixture was stirred under N₂ and heated to 70°C. After 5 hours, sodium cyanoborohydride (59 mg, 0.93 mmol.) was added in one portion, and the reaction mixture was maintained at 70°C for one additional hour. The reaction mixture was cooled, and stored at 0°C overnight.

The reaction mixture was then concentrated in vacuo to give a residue which was re-dissolved in 1:1 H₂O:CH₃CN (5 mL) and HOAc (0.5 mL). The resulting solution was purified by preparatory HPLC (conditions B). The desired fractions, as determined by analytical HPLC (conditions A), were concentrated in vacuo to ∼ 1.5 mL, and desalted. After lyophilization, 1,8-octanediylbis[(oxy-4,1-phenylene)methylene][vancomycin] [A82846B] was obtained (5.2 mg, 0.002 mmol., 7.6 % yield) as a white powder.

HPLC (conditions A) retention time: 14.5 min.

FABMS shows peak of (M+6H) at 3364.

### Preparations 5 & 6:

### Synthesis of Example 47, N⁴-(4-(8-(p-formylphenoxy)-n-octyloxy)benzyl)A82846B, and Example 21, 1,8-octanediylbis[(oxy-4,1-phenylene)methylene]bis[A82846B]

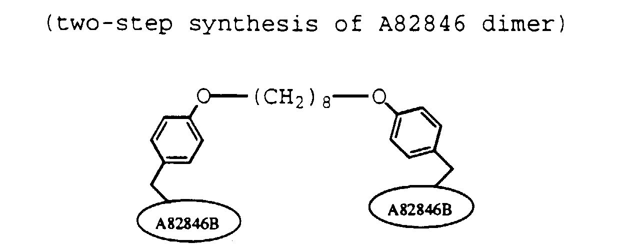

A dry flask was charged with A82846B·tri-acetate salt (5.0 g, 0.003 mol.), and 1,8-bis(4'-formylphenoxy)-n-octane (1.93 g, 0.006 mol.). Anhydrous DMF (300 mL) and anhydrous MeOH (300 mL) were added to the flask and the resulting mixture was stirred under N₂ and heated to 70°C. After 3.75 hours, sodium cyanoborohydride (0.76 g, 0.012 mol.) was added in one portion, and the reaction mixture was maintained at 70°C for one additional hour. The reaction was cooled and stored at 0°C overnight.

The reaction mixture was then concentrated in vacuo to give a residue which was re-dissolved in 1:1 H₂O:CH₃CN (200 mL) and HOAc (5 mL). The resulting solution was purified by preparatory HPLC (conditions B). The desired fractions, as determined by analytical HPLC (conditions A), were concentrated in vacuo to ∼ 1.5 mL, and desalted. After lyophilization, N⁴-(4-(8-(p-formylphenoxy)-n-octyloxy)benzyl)A82846B was obtained (387.4 mg, 0.2 mmol., 6.6 % yield) as a white powder.

HPLC (conditions A) retention time: 19.9 min.

FABMS shows peak of (M+3H) at 1932.

A dry flask was charged with N⁴-(4-(8-(p-formylphenoxy)-n-octyloxy)benzyl)A82846B (20.0 mg, 0.01 mmol), and A82846B (32.9 mg, 0.021 mmol). Anhydrous DMF (3 mL) and anhydrous MeOH (3 mL) were added to the flask and the resulting mixture was stirred under N₂ and heated to 70°C. After 2 hours, sodium cyanoborohydride (5.0 mg, 0.079 mmol) was added in one portion, and the reaction mixture stirred an additional 0.25 hours.

The reaction mixture was then concentrated in vacuo to give a residue which was redissolved in 1:1 H₂O:CH₃CN (5 mL). The resulting solution was purified by preparatory HPLC (conditions D). The desired fraction, as determined by analytical HPLC (conditions A), were concentrated in vacuo to ∼1.5 mL, and desalted. After lyophilization, 1,8-octanediylbis[(oxy-4,1-phenylene)methylene]bis[A82846B] was obtained (3.0 mg, 0.001 mmol, 8.6 % yield) as a white powder.

HPLC (conditions A) retention time:

FABMS shows peak of (M+5H) at 3508.

### Preparations 7 & 8:

### Synthesis of Example 70, 1,3-phenylenebis[oxy-1,3-n-propylene-oxy-4,1-phenylene)methylene]A82846B/A82846B, (3-dimethylaminopropyl)amide, and Example 71, 1,3-phenylenebis[oxy-1,3-n-propylene-oxy-4,1-phenylene)methylene]bis[A82846B, (3-dimethylaminopropyl)amide]

A dry round bottom flask was charged with 1,3-phenylene-bis-[(oxy-1,3-n-propyleneoxy-4,1-phenylene)methylene]--bis[A82846B] (50.0 mg, 0.014 mmol) and 1 mL DMSO. PyBOP (14.5 mg, 0.028 mmol) and 3-dimethylaminopropylamine (2.8 mg, 0.028 mmol) were added and the reaction was stirred at room temperature under nitrogen for one hour. The reaction mixture was then concentrated in vacuo to give a residue which was re-dissolved in 1:1 H₂O:CH₃CN (5 mL). The resulting solution was purified by preparatory HPLC (conditions B). The desired fractions, as determined by analytical HPLC (conditions A) were concentrated in vacuo to - 1.5 mL, and desalted as in previous examples. After lyopholization 1,3-phenylenebis[oxy-1,3-n-propylene-oxy-4,1-phenylene)methylene]bis[A82846B, (3-dimethylaminopropyl)amide] (6.9 mg, 13.1% yield) and 1,3phenylenebis [oxy-1,3-n-propylene-oxy-4,1-phenylene)-methylene]A82846B/A82846B,(3-dimethylaminopropyl)amide (6.6 mg, 12.8% yield) were obtained as white powders.
1,3-phenylenebis[oxy-1,3-n-propylene-oxy-4,1-phenylene)methylene]bis[A82846B, (3-dimethylaminopropyl)amide]
HPLC (conditions A) retention time: 13.2 min.
FABMS shows peak of (M+9H) at 3761.
1,3-phenylenebis [oxy-1,3-n-propylene-oxy-4,1-phenylene)-methylene]A82846B/A82846B,(3-dimethylaminopropyl)amide HPLC (conditions A) retention time: 13.7 min. FABMS shows peak of (M+6H) at 3674.

Details concerning the synthesis of all of the compounds of TABLES 1 and 2, as well as identifying characteristics on the same compounds, are presented in TABLES 3 and 4.

**TABLE 3**

| Ex. # | Aldehyde | HPLC *Retention Minutes | % yield | FAB•MS M/Z | M+x H |
|---|---|---|---|---|---|
| 1 | 1,2-bis(4-formylphenoxy)-n-ethane | 11.5 | 1.86 | 3136 | 3 |
| 2 | 1,4-bis(2-formylphenoxy)-n-butane | 11.8 | 0.79 | 3165 | 4 |
| 3 | 1,5-bis(4-formylphenoxy)-n-pentane | 12.9 | 5.42 | 3178 | 4 |
| 4 | 1,5-bis(3-formylphenoxy)-n-pentane | 13.0 | 4.08 | 3179 | 4 |
| 5 | 1,6-bis(4-formylphenoxy)-n-hexane | 13.6 | 9.05 | 3195 | 6 |
| 6 | 3-methyl-1,5-bis(4-formylphenoxy)-n-pentane | 13.5 | 4.54 | 3193 | 4 |
| 7 | 1,7-bis(4-formylphenoxy)-n-heptane | 14.7 | 5.00 | 3207 | 5 |
| 8 | 1,8-bis(4-formylphenoxy)-n-octane | 15.5 | 3.91 | 3219 | 2 |
| 9 | 1,9-bis(4-formylphenoxy)-n-nonane | 16.4 | 4.41 | 3235 | 4 |
| 10 | 1,2-bis(2-(4-formylphenoxy)-ethoxy)ethane | 12.3 | 1.89 | 3226 | 4 |
| 11 | 1,4-bis(2-(p-formylphenoxy)-ethoxy)carbonyl-benzene | 13.0 | 10.50 | 3331 | 6 |
| 12 | 1,3-bis(3-(p-formylphenoxy)-n-propyloxy)-benzene | 15.5 | 3.10 | 3300 | 4 |
| 13 | 1,8-bis(4-formylphenoxy)-n-octane | 14.5 | 5.95 | 3364 | 4 |
| 14 | 1,3-bis(4-formylphenoxy)-propane | 9.4 | 14.29 | 3436 | 4 |
| 15 | 1,4-bis(2-formylphenoxy)-n-butane | 10.2 | 5.91 | 3452 | 5 |
| 16 | 1,5-bis(4-formylphenoxy)-n-pentane | 10.4 | 3.86 | 3466 | 5 |
| 17 | 1,5-bis(3-formylphenoxy)-n-pentane | 11.3 | 22.41 | 3465 | 4 |
| 18 | 1,6-bis(4-formylphenoxyl-n-hexane | 11.3 | 5.46 | 3478 | 4 |
| 19 | 3-methyl-1,5-bis(4-formylphenoxy)-n-pentane | 11.3 | 8.14 | 3479 | 4 |
| 20 | 1,7-bis(4-formylphenoxy)-n-heptane | 12.5 | 5.73 | 3494 | 5 |
| 21 | 1,8-bis(4-formylphenoxy)-n-octane | 14.0 | 13.31 | 3508 | 5 |
| 23 | 1,8-bis(3-formylphenoxy)-n-octane | 14.4 | 21.23 | 3508 | 5 |
| 24 | 1,8-bis(4-formyl-2-n-pentyloxy-phenoxy)-n-octane | 20.8 | 16.91 | 3680 | 5 |
| 25 | 1,9-bis(4-formylphenoxy)-n-nonane | 14.9 | 9.31 | 3522 | 5 |
| 26 | 1,10-bis(4-formylphenoxy)-n-decane | 15.9 | 8.87 | 3535 | 5 |
| 27 | 1,12-bis (4-formylphenoxy)-n-dodecane | 17.7 | 1.32 | 3565 | 6 |
| 28 | 1,16-bis(4-formylphenoxy)-n-hexadecane | 20.4 | 4.05 | 3625 | 9 |
| 29 | 1,2-bis(2-(4-formylphenoxy)-ethoxy)ethane | 10.2 | 6.28 | 3511 | 4 |
| 30 | 1,3-bis(3-(p-formylphenoxy)-n-propyloxy)-benzene | 15.2 | 22.96 | 3589 | 5 |
| 31 | 1,4-bis(2-(p-formylphenoxy)-ethoxy)carbonyl-benzene | 11.7 | 24.94 | 3615 | 5 |
| 32 | 5-phenyl-1,3-bis(3-(p-formylphenoxy)-n-propyloxy)-benzene | 16.8 | 12.88 | 3664 | 5 |
| 49 | 1,6-bis(4-(4-formylphenyl)-phenoxy)hexane | 16.2 | 11.58 | 3633 | 5 |
| 50 | 1,3-bis(5-(4-formylphenoxy)-n-pentyloxy)-benzene | 17.0 | 9.31 | 3645 | 6 |
| 51 | 1,8-bis(2-phenyl-5-formylphenoxy)-octane | 18.3 | 10.83 | 3662 | 6 |
| 53 | 1,8-bis(3-formylphenoxy)-n-hexane | 15.3 | 2.1 | 3221 | 4 |
| 54 | 1,6-bis(4-(4'-formylphenoxy)-phenoxy)-n-hexane | 18.2 | 6.1 | 3347 | 6 |
| 55 | 1,8-bis(3-formyl-2-iodophenoxy)-n-hexane | 22.9 | 2.2 | 3471 | 3 |
| 56 | 1,8-bis(2-phenyl-5-formylphenoxy)-n-octane | 19.3 | 2.8 | 3374 | 4 |
| 57 | 1,3-bis(6-(2-dimethyl)-1-hexanaloxy)-benzene | 15.5 | 8.2 | 3229 | 4 |
| 58 | 1,4-bis(6-(2-dimethyl)-1-hexanaloxy)-butane | 13.5 | 6.1 | 3209 | 4 |
| 59 | 1, 12-bis(4-formylphenoxy)-n-dodecane | 21.6 | 6.8 | 3278 | 5 |
| 60 | 1,3-bis(3-(-formylphenoxy-n-propyloxy)-benzene | HCL SALT | | | |
| 61 | 1,3-bis(3-(p-formylphenoxy-n-propyloxy)-5-n-pentylbenzene | 36.6 | 12.7 | 3660 | 8 |
| 62 | 1,8-bis(3-formyl-2-iodophenoxy)-n-octane | 17.1 | 5.4 | 3762 | 6 |
| 63 | 1,3-bis(6-(2-dimethyl)-1-hexanaloxy)-benzene | 13.3 | 15.5 | 3516 | 5 |
| 64 | 1,3-bis(3-(p-formylphenoxy-n-heptyloxy)-benzene | 19.3 | 1.4 | 3701 | 6 |
| 65 | 1,4-bis(6-(2-dimethyl)-1-hexanaloxy)-butane | 13.5 | 24.8 | 3495 | 4 |
| 66 | 1,3-bis(3-(p-formylphenoxy-n-propyloxy)-benzene | HCL SALT | | | |
| 70 | 1,3-bis(3-(p-formylphenoxy)-n-propyloxy)-benzene | 13.7 | 12.8 | 3674 | 6 |
| 71 | 1,3-bis(3-(p-formylphenoxy)-n-propyloxy)-benzene | 13.2 | 13.1 | 3761 | 9 |

| | | | | | |
|---|---|---|---|---|---|
| * Conditions A | | | | | |

**TABLE 4**

| Ex. # | Aldehyde | HPLC *Retention, Minutes | % Yield | FAB•MS M/Z | M+ xH |
|---|---|---|---|---|---|
| 33 | 1,2-bis(4-formylphenoxy)-n-ethane | 13.1 | 2.58 | 1706 | 5 |
| 34 | 1,2-bis(4-formylphenoxy)-n-propane | 15.2 | 13.08 | 1718 | 0 |
| 35 | 1,2-bis(4-formylphenoxy)-n-butane | 14.7 | 8.12 | 1734 | 4 |
| 36 | 1,2-bis(4-formylphenoxy)-n-pentane | 17.1 | 21.89 | 1746 | 4 |
| 37 | 1,2-bis(4-formylphenoxy)-n-hexane | 18.0 | 8.37 | 1760 | 2 |
| 38 | 1,2-bis(4-formylphenoxy)-3-methyl-n-pentane | 17.7 | 5.81 | 1760 | 1 |
| 39 | 1,2-bis(4-formylphenoxy)-n-heptane | 19.0 | 27.99 | 1774 | 2 |
| 40 | 1,2-bis(4-formylphenoxy)-n-octane | 18.5 | 11.89 | 1789 | 3 |
| 41 | 1,2-bis(4-formylphenoxy)-n-decane | 21.7 | 0.85 | 1816 | 4 |
| 42 | 1,2-bis(4-formylphenoxy)-n-dodecane | 23.4 | 12.53 | 1841 | 1 |
| 43 | 1,2-bis(2-(4-formylphenoxy)ethoxyethane | 14.4 | 5.57 | 1794 | 4 |
| 44 | 1,2-bis(2-(4-formylphenoxy)ethoxybenzoate | 16.5 | 5.64 | 1897 | 3 |
| 45 | 1,2-bis(2-(4-formylphenoxy)-propyloxy)-5-phenylbenzene | 21.0 | 4.96 | 1946 | 5 |
| 46 | 1,3-bis(4-formylphenoxy)-propane | 15.2 | 4.90 | 1861 | 2 |
| 47 | 1,3-bis(4-formylphenoxy)-octane | 19.9 | 8.69 | 1930 | 1 |
| 48 | 1,3-bis(4-formylphenoxy)-dodecane | 23.3 | 1.19 | 1984 | 1 |
| 52 | 1,3-bis(3-(p-formylphenoxy)-n-propyloxy)-benzene | 18.8 | 3.10 | 1871 | 5 |
| 67 | 1,8-bis(3-formyl-2-iodophenoxy)-n-octane | 21.3 | 4.5 | 2042 | 5 |
| 68 | 1,3-bis(6-(2-dimethyl)-1-hexanaloxy)-benzene | 18.3 | 13.2 | 1798 | 4 |
| 69 | 1,3-bis(3-(p-formylphenoxy-n-propyloxy)-5-n-pentylbenzene | 22.4 | 3 | 1940 | 5 |

| | | | | | |
|---|---|---|---|---|---|
| * Conditions A | | | | | |

The compounds of Formulae I and II are useful for the treatment of bacterial infections. Therefore, in another embodiment, the present invention is directed to a method for controlling a bacterial infection in a host animal, typically a warm-blooded animal, which comprises administering to the host animal an effective, antibacterial amount of a compound of Formula I or II. In this embodiment, the compounds of the present invention can be used to control and treat infections due to various bacteria, but especially gram-positive bacteria. In a preferred embodiment, the compounds are used to control and treat infections due to bacteria resistant to existing antibacterials. For example, certain bacteria are resistant to methicillin, and yet others are resistant to vancomycin and/or teicoplanin. The present compounds provide a technique for controlling and treating infections due to such resistant bacterial species.

In carrying out this embodiment of the invention, the compounds can be administered by any of the conventional techniques, including the oral route and parenteral routes such as intravenous and intramuscular. The amount of compound to be employed is not critical and will vary depending on the particular compound employed, the route of administration, the severity of the infection, the interval between dosings, and other factors known to those skilled in the art. In general, a dose of from about 0.5 to about 100 mg/kg will be effective; and in many situations, lesser doses of from about 0.5 to about 50 mg/kg will be effective. A compound of the present invention can be administered in a single dose, but in the known manner of antibacterial therapy, a compound of the present invention is typically administered repeatedly over a period of time, such as a matter of days or weeks, to ensure control of the bacterial infection.

Also in accordance with known antibacterial therapy, a compound of the present invention is typically formulated for convenient delivery of the requisite dose. Therefore, in another embodiment, the present invention is directed to a pharmaceutical formulation comprising a compound of either Formula I or II, in combination with a pharmaceutically-acceptable carrier. Such carriers are well known for both oral and parenteral routes of delivery. In general, a formulation will comprise a compound of the present invention in a concentration of from about 0.1 to about 90% by weight, and often from about 1.0 to about 3%.

The antibacterial efficacy of the present compounds is illustrated by following TABLES 5 and 6. The minimal inhibitory concentrations (MICs) were determined using a standard broth micro-dilution assay. TABLE 6 presents a comparison of the activity of illustrative compounds against representative vancomycin-resistant and vancomycin-sensitive enterococci (Enterococcus faecium and Enterococcus faecalis, mean geometric MIC (mcg/mL), as determined by the standard broth micro-dilution assay.

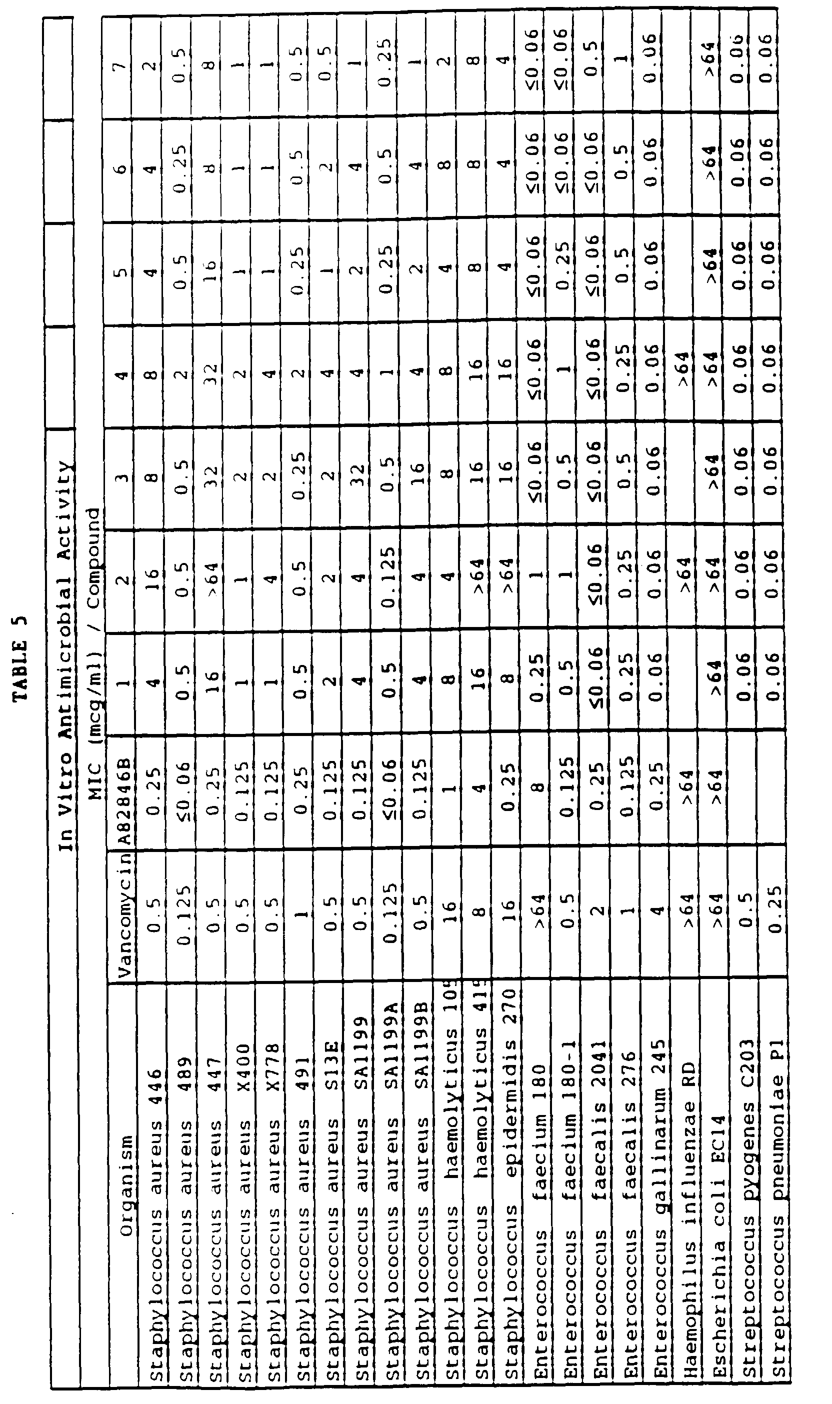

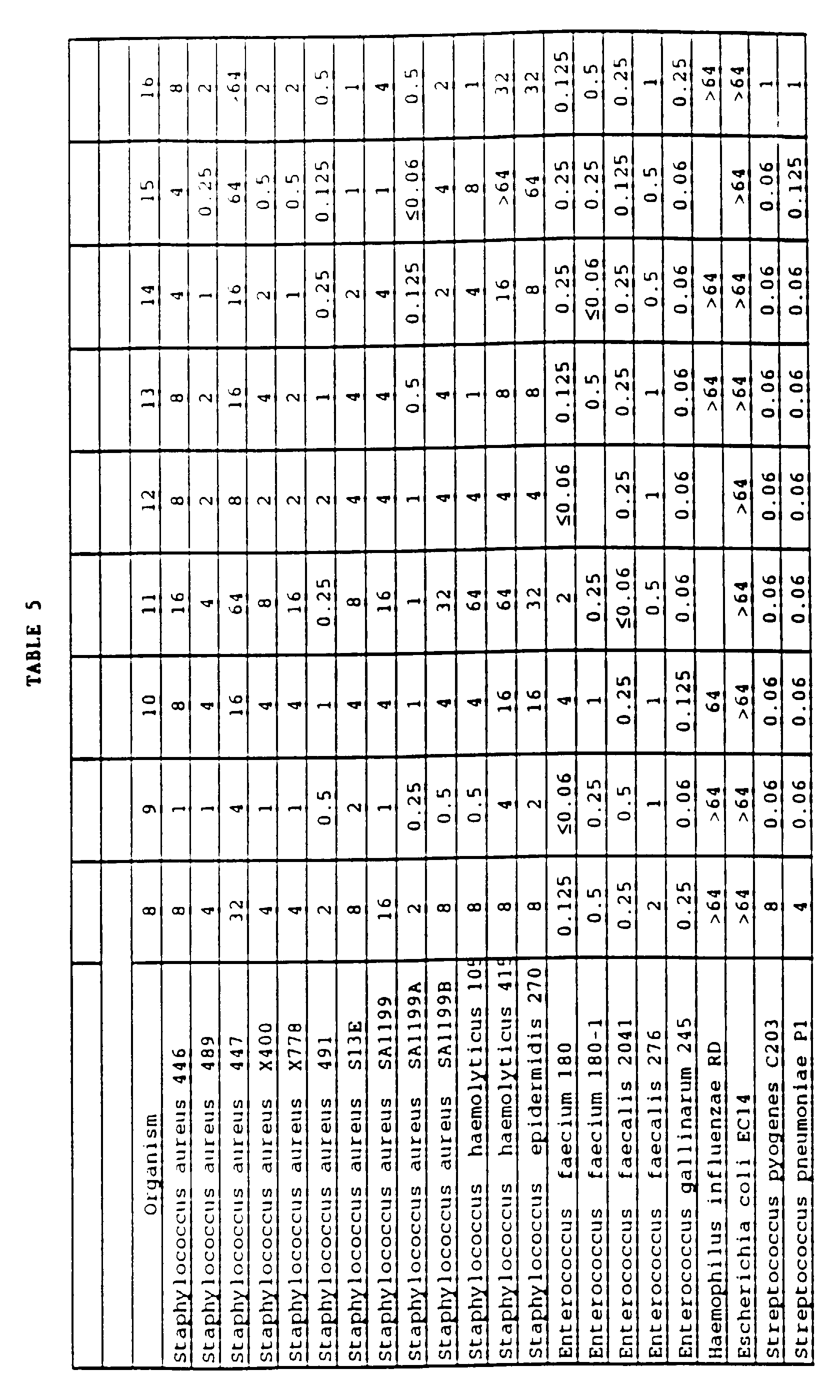

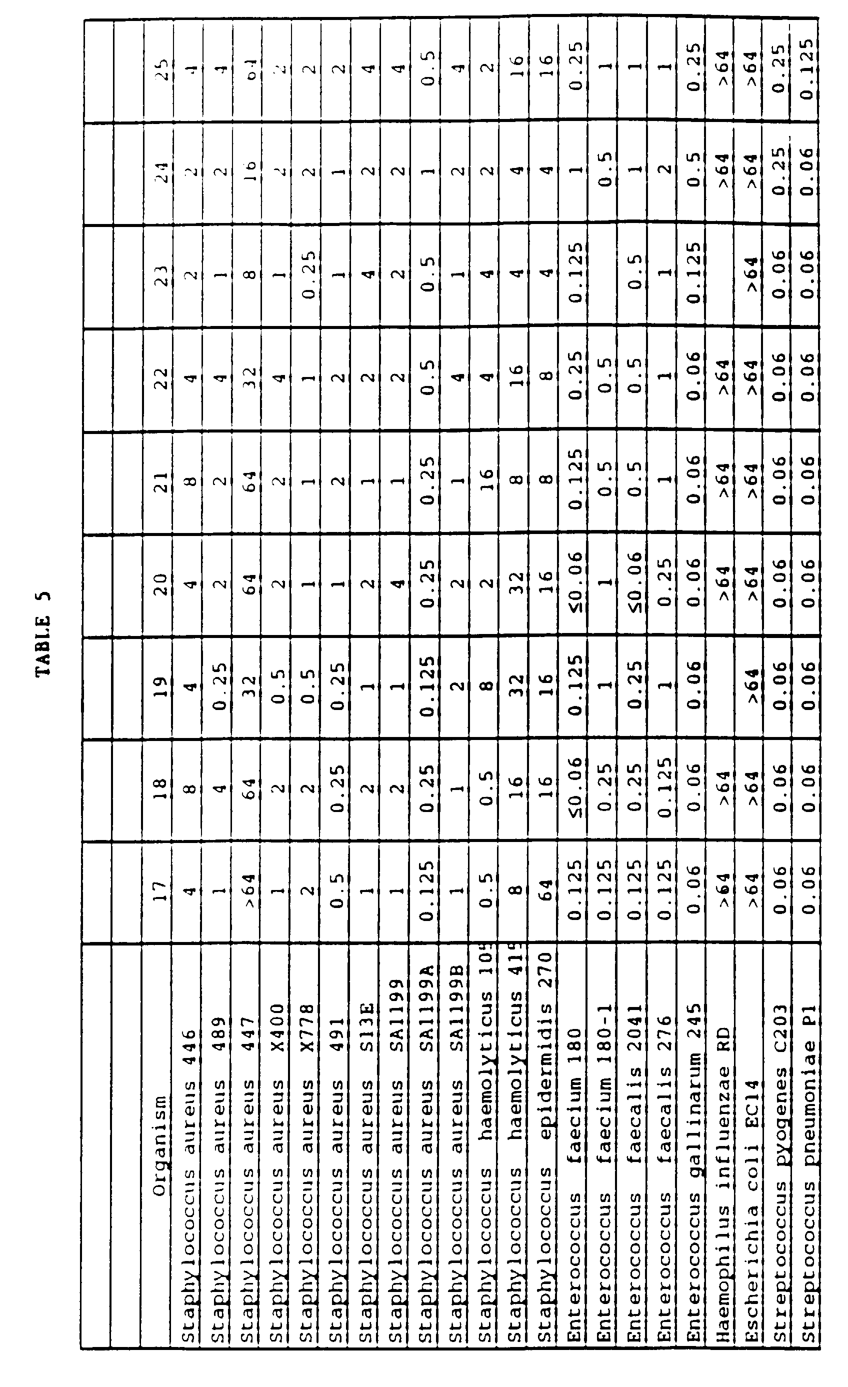

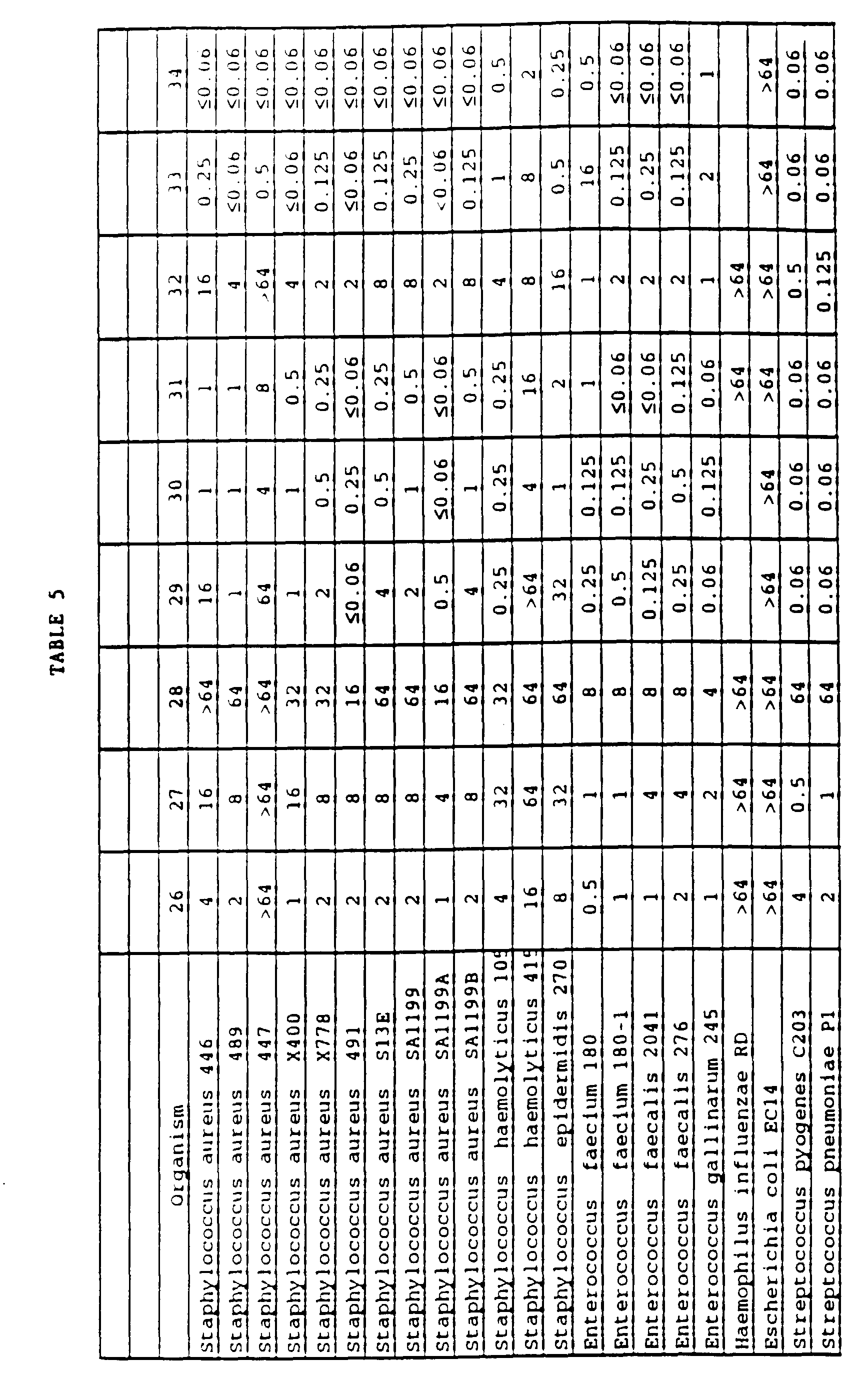

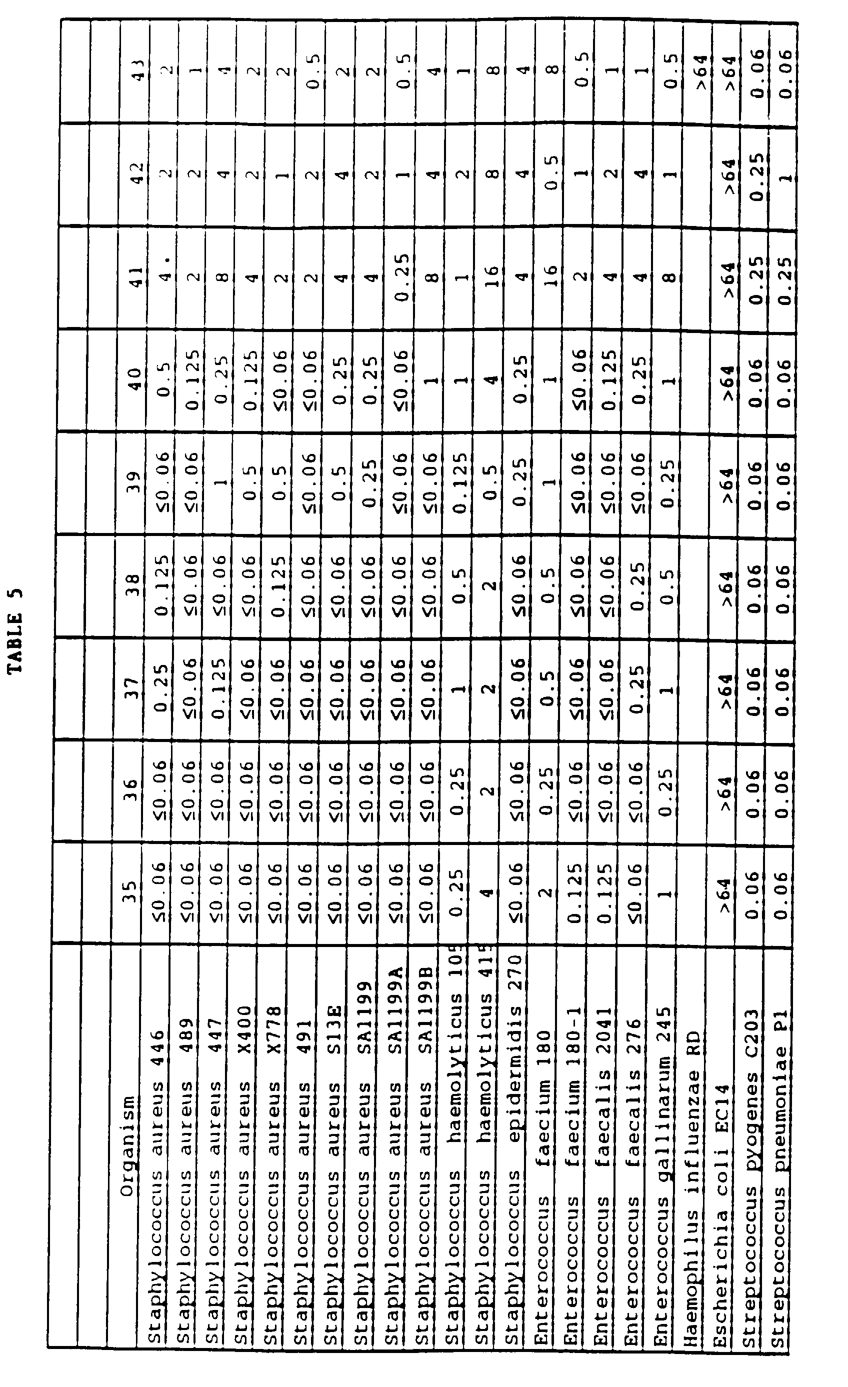

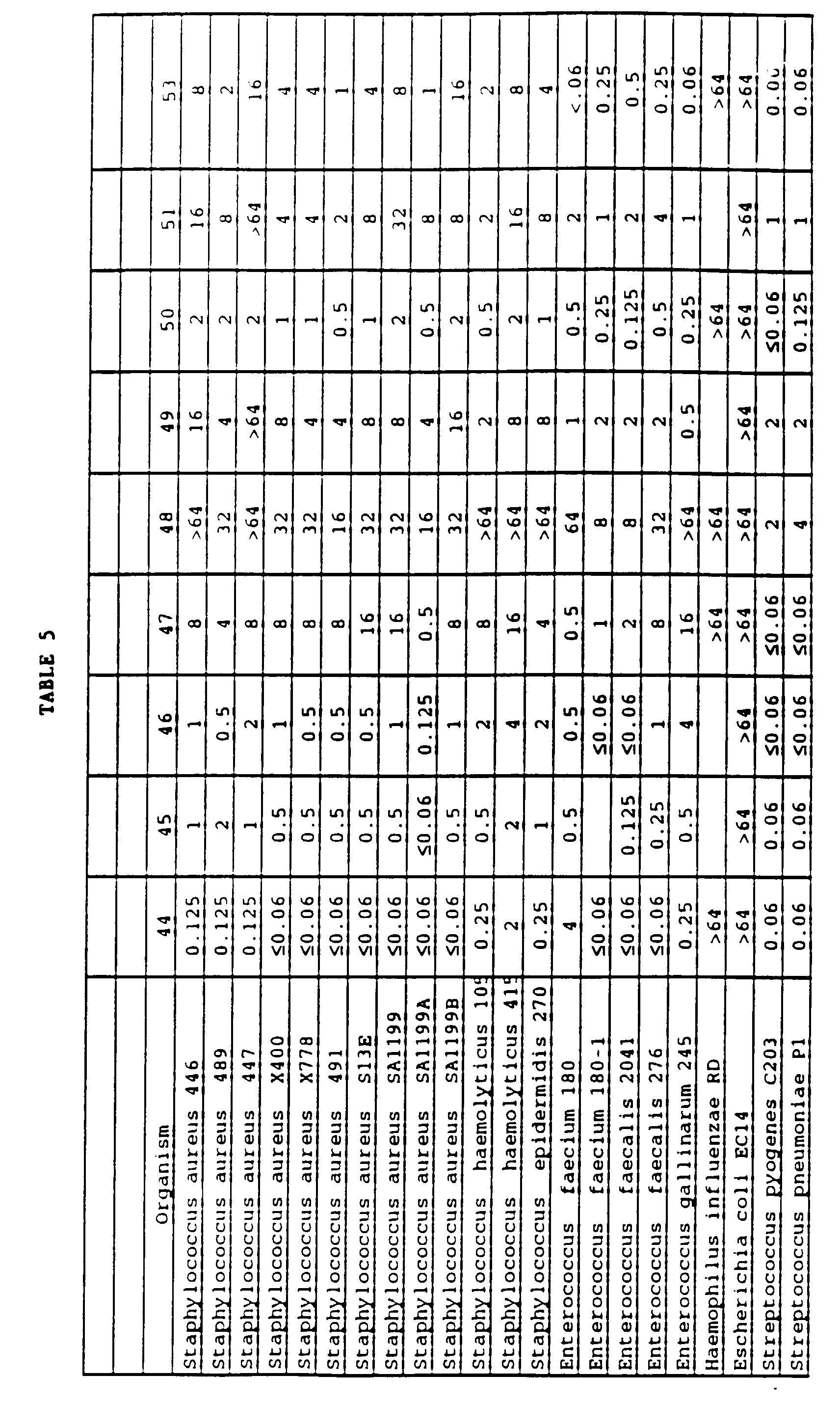

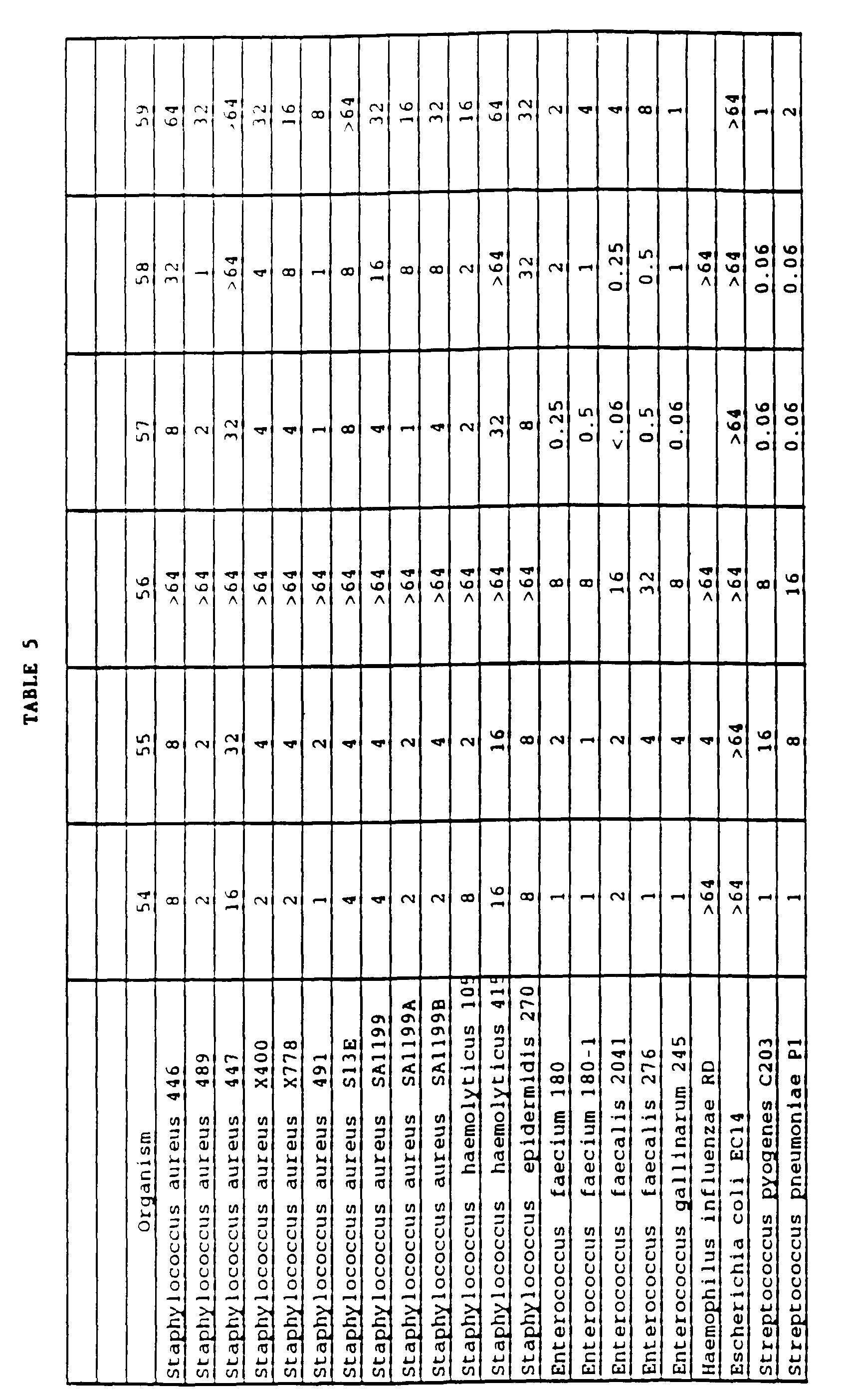

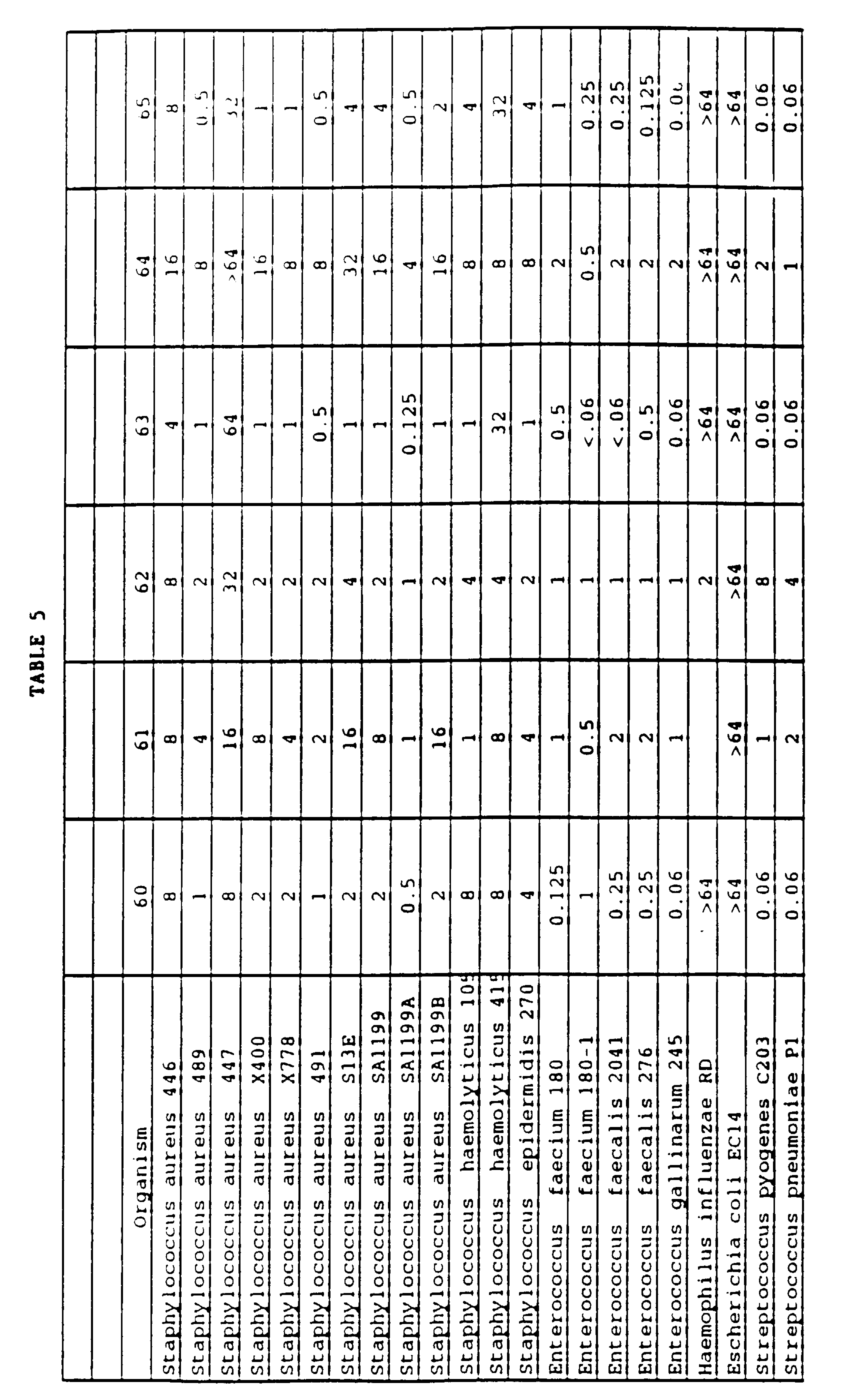

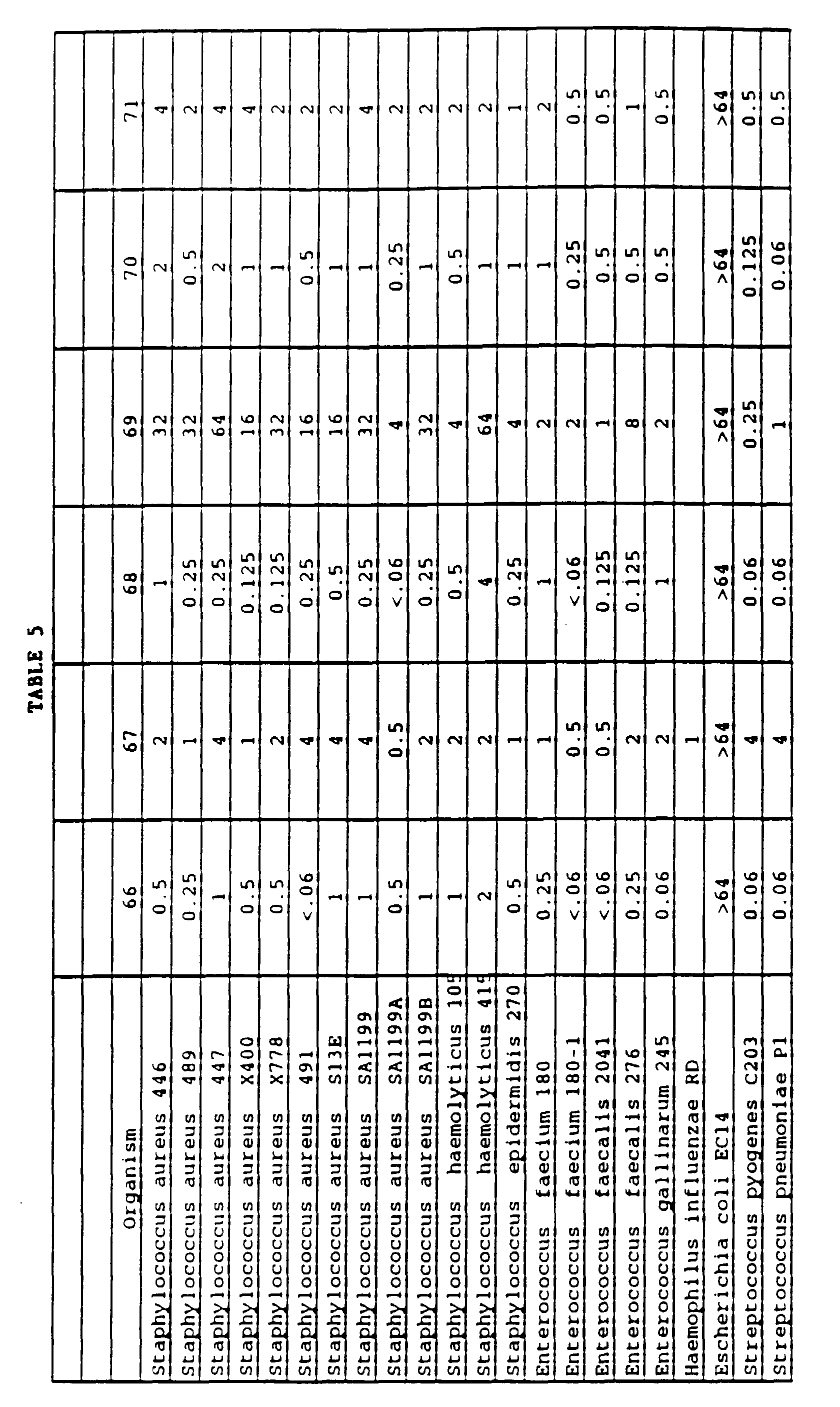

**TABLE 6**

| **In Vitro Activity Against Enterococci** | | |
|---|---|---|
| Cpd. Number | Vancomycin Resistant Strains | Vancomycin Sensitive Strains |
| Vancomycin | 282 | 3.9 |
| A282846B | 29 | 0.22 |
| 1 | 42 | 1.3 |
| 2 | 27 | 1.0 |
| 3 | 27 | 1.5 |
| 4 | 19 | 2.0 |
| 5 | 11 | 0.87 |
| 6 | 32 | 1.3 |
| 7 | 8.0 | 1.3 |
| 8 | 9.5 | 0.87 |
| 9 | 9.5 | 1.2 |
| 10 | >90 | 3.0 |
| 11 | 38 | 1.7 |
| 12 | 4.0 | 0.66 |
| 13 | 9.5 | 1.2 |
| 14 | >64 | 1.0 |
| 15 | 23 | 0.87 |
| 16 | 38 | 1.5 |
| 17 | 4.8 | 0.57 |
| 18 | 19 | 1.0 |
| 19 | 19 | 0.76 |
| 20 | 13 | 1.0 |
| 21 | 2.8 | 0.87 |
| 22 | 6.7 | 0.76 |
| 23 | 1.7 | 0.5 |
| 24 | 4.8 | 1.2 |
| 25 | 6.7 | 1.2 |
| 26 | 4.0 | 1.5 |
| 27 | 3.4 | 1.7 |
| 28 | 9.5 | 6.1 |
| 29 | 38 | 1.3 |
| 30 | 1.7 | 0.38 |
| 31 | 27 | 0.66 |
| 32 | 2.8 | 1.5 |
| 33 | >128 | 1.3 |
| 34 | 53 | 0.87 |
| 35 | >81 | 0.57 |
| 36 | 6.7 | 0.29 |
| 37 | 9.5 | 9.1 |
| 38 | 4.8 | 1.1 |
| 39 | 5.7 | 1.0 |
| 40 | 11.3 | 0.19 |
| 41 | 3.4 | 1.1 |
| 42 | 4.5 | 1.1 |
| 43 | >128 | 4.6 |
| 44 | 76 | 0.66 |
| 45 | 2.8 | 0.33 |
| 46 | 4.8 | 0.17 |
| 47 | 2.8 | 1.0 |
| 48 | 8.0 | 5.2 |
| 49 | 2.0 | 1.5 |
| 50 | 1.7 | 0.44 |
| 51 | 3.4 | 2.3 |
| 52 | 4.8 | 0.093 |
| 53 | 5.7 | 0.66 |
| 54 | 4.8 | 2.3 |
| 55 | 4.8 | 2.3 |
| 56 | 8 | 5.3 |
| 57 | 11.3 | 0.76 |
| 58 | 64 | 1 |
| 59 | 2.4 | 1.2 |
| 60 | 2.4 | 1.3 |
| 61 | 2 | 1.2 |
| 62 | 3.4 | 1.5 |
| 63 | 4.8 | 0.57 |
| 64 | 6.7 | 4 |
| 65 | 22 | 0.57 |
| 66 | 2 | 0.38 |
| 67 | 3.4 | 0.66 |
| 68 | 9.5 | 0.25 |
| 69 | 5.7 | 2.3 |
| 70 | 3.4 | 0.38 |
| 71 | 2.4 | 0.38 |

## Claims

1. A compound of the formula: 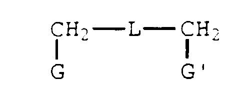 wherein each of G and G' is independently deshydrovancomycin of the formula: 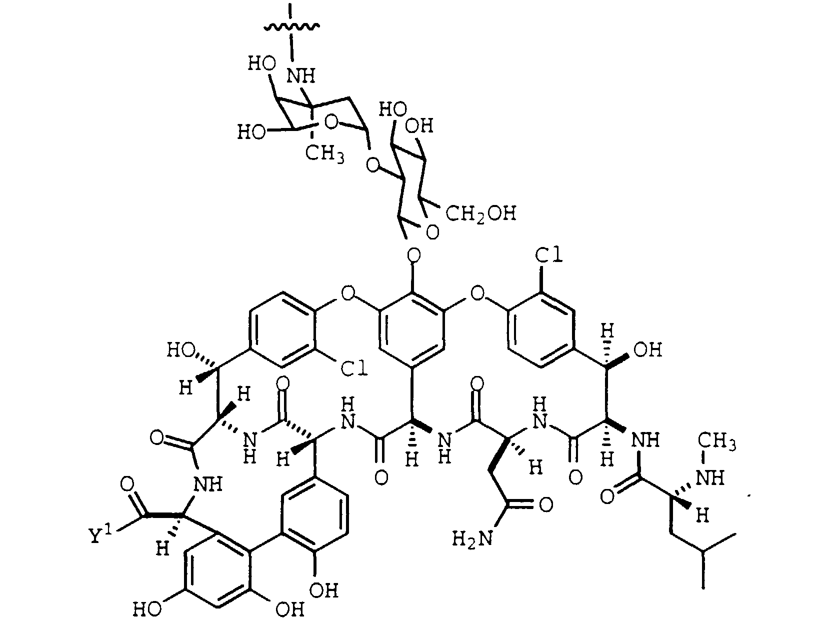 or deshydroA82846B of the formula: 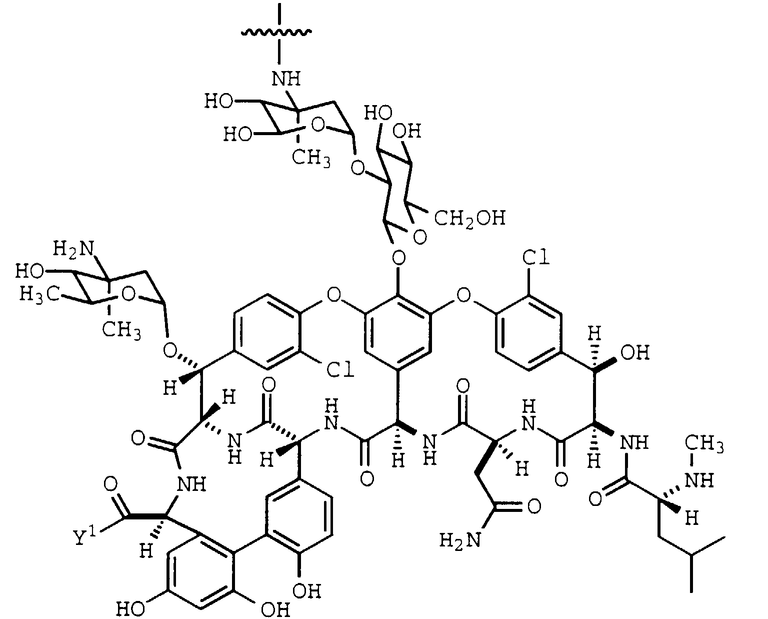 wherein Y¹ is OH or 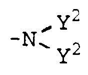 and Y² is defined as follows:
(1) each Y² independently represents
hydrogen,
alkyl of C₁-C₁₀,
cycloalkyl of C₅-C₆.
cycloalkenyl of C₅-C₆,
naphthyl,
biphenylyl,
radical of the formula -Y³-(Y⁴)_{0, 1, or 2}, wherein Y³ is loweralkyl of C₁-C₆ optionally substituted by from one to three substituents, each of which is independently selected from the group consisting of halo, nitro, cyano, alkoxy, haloalkyl, and haloalkoxy; and Y⁴ is 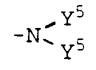 wherein each Y⁵ is independently hydrogen or loweralkyl of C₁-C₄, or Y⁴ is phenyl or phenyl substituted with from one to three substituents, each of which is independently
halo,
nitro,
loweralkyl of C₁-C₄,
cycloalkyl of C₅-C₆,
loweralkoxy of C₁-C₄,
haloloweralkyl of C₁-C₄, or
haloloweralkoxy of C₁-C₄; or
(2) one Y² is hydrogen and the other Y² is (2-furanon-3-yl); or
(3) both Y²s are taken together with the nitrogen and constitute a five- to seven-membered heterocyclic ring optionally containing in addition to the indicated nitrogen atom one additional hetero ring atom which is nitrogen, oxygen, or sulfur, and which heterocyclic radical can be unsubstituted or substituted with from one or two substituents, each of which is loweralkyl of C₁-C₂, loweralkoxy of C₁-C₂, phenyl, benzyl, or C₁-C₆-alkanoyl; and L is a divalent linking radical of the formula A: 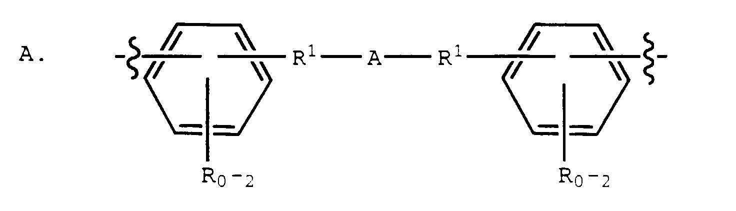 wherein
A is:
alkylene of C₁-C₁₆,
(alkylene of C₁-C₄-X')_{q}-alkylene of C₁-C₄, wherein q is 1-3,
alkylene of 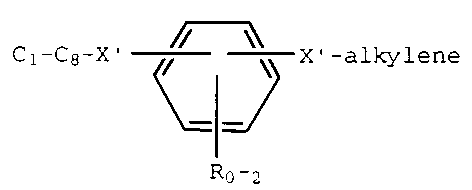 of C₁ -C₈;
alkylene of 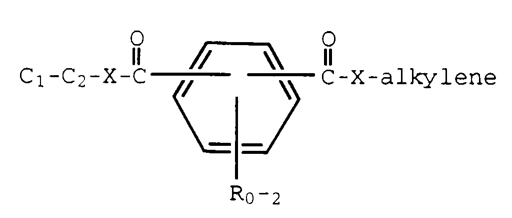 of C₁-C₂, or
alkylene of 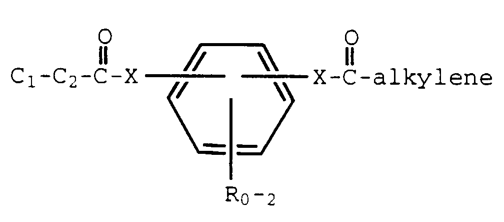 of C₁-C₂ ;
each R¹ is independently
CH₂,
O,
S, 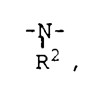 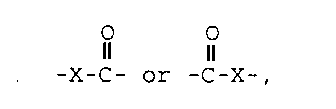 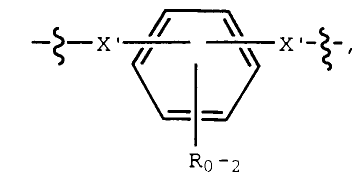 or 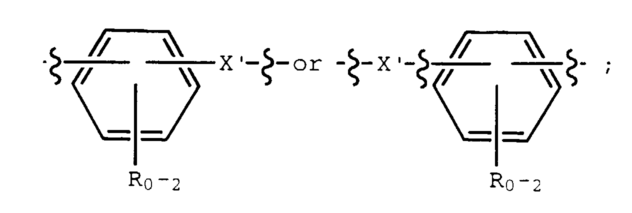 wherein each R independently represents halo, loweralkyl of C₁-C₆, loweralkoxy of C₁-C₆, phenyl, or phenyl substituted by from 1 to 2 substituents, each of which is independently halo, loweralkyl of C₁-C₆, or loweralkoxy of C₁-C₆; each X is independently -O- or 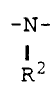
wherein R² is H or loweralkyl of C₁-C₄; and each X' is independently -O-, -S-, or 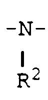 wherein
R² is as defined above; or L is a divalent linking radical of the formula B:
B. -alkylene of C₁-C₈-R³-X"-R³-alkylene of C₁-C₈- wherein X" represents alkylene of C₁-C₄ or a phenylene of the formula 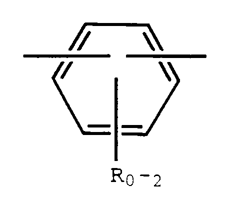 wherein R is as defined above; and each R³ is independently CH₂ or O, or a salt thereof.

2. A compound of Claim 1 wherein both of G and G' are deshydro A82846B.

3. A compound of Claim 1 or 2 wherein L is a linking radical of formula A, A is alkylene of C₁-C₁₆, and both R¹ are O.

4. A compound of Claim 3 wherein A is straight-chain alkylene of C₆-C₁₂.

5. A compound of Claim 1 or 2 wherein L is a linking radical of formula A, A is (alkylene of C₁-C₄-X')_{q}-alkylene of C₁-C₄, q=2, and both R¹ are O.

6. A compound of the formula:
G―CH₂―L―CHO
wherein G is selected from the group consisting of deshydrovancomycin of the formula: 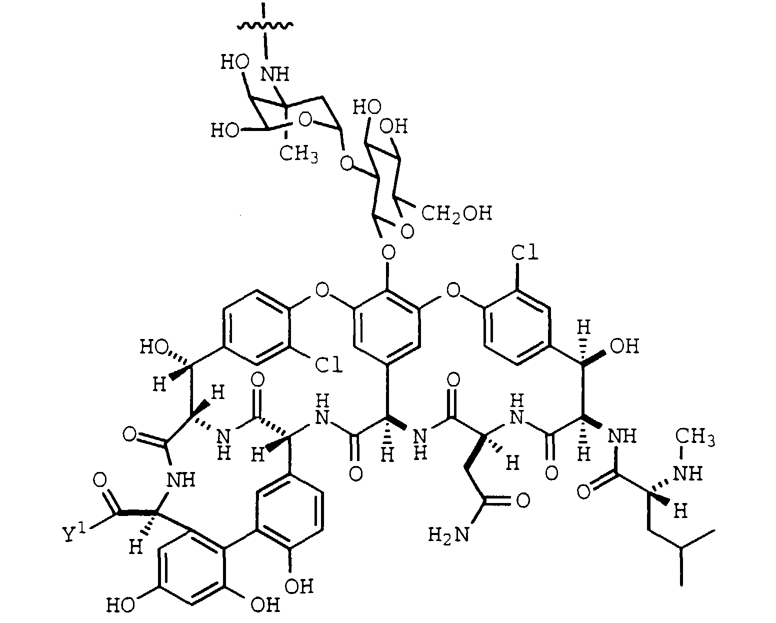 and deshydroA82846B of the formula: 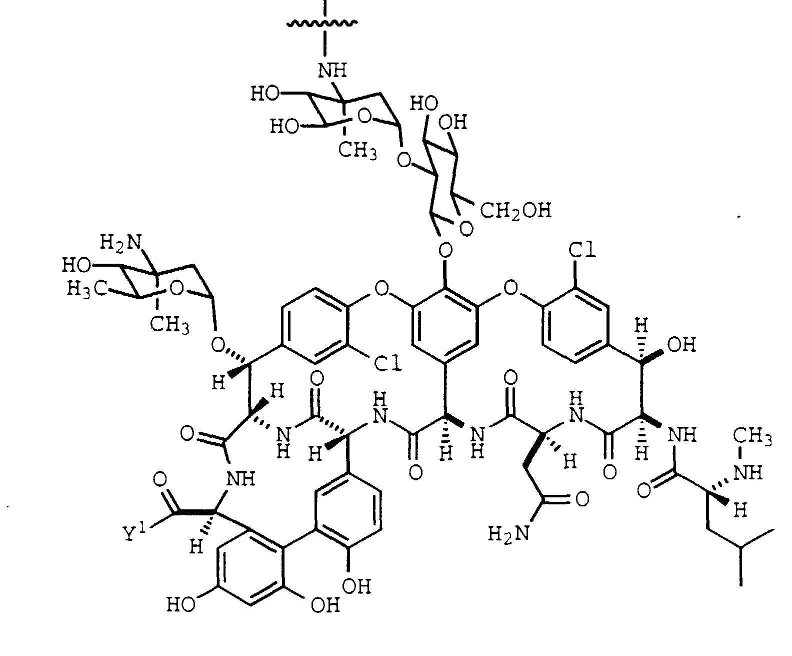 wherein Y¹ is OH or 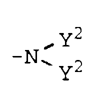 and Y² is defined as follows:
(1) each Y² independently represents
hydrogen,
alkyl of C₁-C₁₀,
cycloalkyl of C₅-C₆.
cycloalkenyl of C₅-C₆,
naphthyl,
biphenylyl,
radical of the formula -Y³-(Y⁴)_{0, 1, or 2}, wherein Y³ is loweralkyl of C₁-C₆ optionally substituted by from one to three substituents, each of which is independently selected from the group consisting of halo, nitro, cyano, alkoxy, haloalkyl, and haloalkoxy; and Y⁴ is 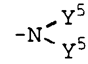 wherein each Y⁵ is independently hydrogen or loweralkyl of C₁-C₄, or Y⁴ is phenyl or phenyl substituted with from one to three substituents, each of which is independently
halo,
nitro,
loweralkyl of C₁-C₄,
cycloalkyl of C₅-C₆,
loweralkoxy of C₁-C₄,
haloloweralkyl of C₁-C₄, or
haloloweralkoxy of C₁-C₄; or
(2) one Y² is hydrogen and the other Y² is (2-furanon-3-yl); or
(3) both Y²s are taken together with the nitrogen and constitute a five- to seven-membered heterocyclic ring optionally containing in addition to the indicated nitrogen atom one additional hetero ring atom which is nitrogen, oxygen, or sulfur, and which heterocyclic radical can be unsubstituted or substituted with from one or two substituents, each of which is loweralkyl of C₁-C₂, loweralkoxy of C₁-C₂, phenyl, benzyl, or C₁-C₆-alkanoyl; and L is a divalent linking radical of the formula A: 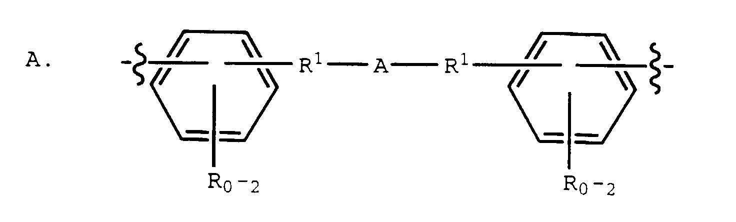
wherein
A is:
alkylene of C₁-C₁₆,
(alkylene of C₁-C₄-X')_{q}-alkylene of C₁-C₄, wherein q is 1-3,
alkylene of 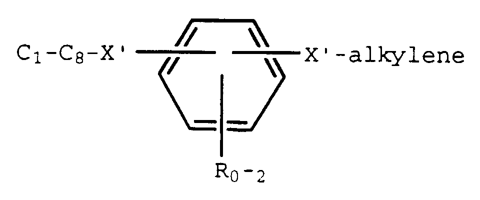 of C₁ -C₈;
alkylene of 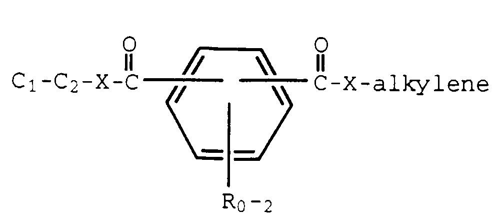 of C₁ -C₂, or
alkylene of 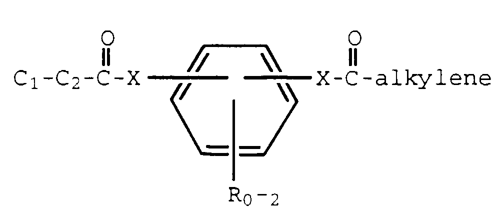 of C₁ -C₂;
each R¹ is independently
CH₂,
O,
S, 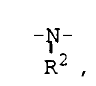 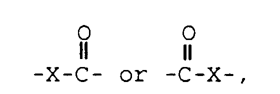 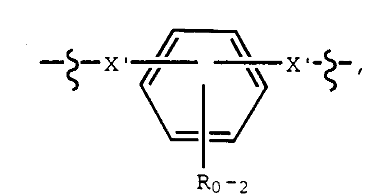 or 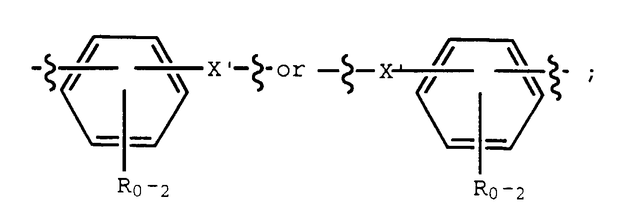 wherein each R independently represents halo, loweralkyl of C₁-C₆, loweralkoxy of C₁-C₆, phenyl, or phenyl substituted by from 1 to 2 substituents, each of which is independently halo, loweralkyl of C₁-C₆, or loweralkoxy of C₁-C₆; each X is independently -O- or 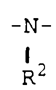 wherein R² is H or loweralkyl of C₁-C₄; and each X' is independently -O-, -S-, or 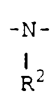 wherein
R² is as defined above; or L is a divalent linking radical of the formula B:
B. -alkylene of C₁-C₈-R³-X"-R³-alkylene of C₁-C₈-
wherein X" represents alkylene of C₁-C₄ or a phenylene of the formula 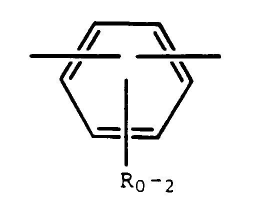 wherein R is as defined above; and each R³ is independently CH₂ or O, or a salt thereof.

7. A pharmaceutical formulation comprising a compound of Claim 1 or 6 in combination with a pharmaceutically-acceptable diluent or carrier.

8. A compound according to any one of claims 1 to 6 for the use as a pharmaceutical.

9. A compound according to any one of claims 1 to 6 for the use in the treatment of a bacterial infection in a host.

10. A compound for the use according to claim 9, wherein the bacterial infection is attributable to a vancomycin-resistant-enterococcus.

11. A compound of claim 1 or 6 for use in antibacterial therapy.

12. A compound of claim 1 or 6 for use in antibacterial therapy against vancomycin-resistant-enterococcus.

13. A process for the preparation of a compound as claimed in any one of claims 1-6 which comprises reducing a Schiff base corresponding to the desired compound of claims 1-6, and if desired, thereafter forming a pharmaceutically acceptable salt.

## Patentansprüche

1. Verbindung der Formel 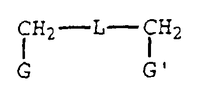 worin G und G' jeweils unabhängig steht für Deshydrovancomycin der Formel 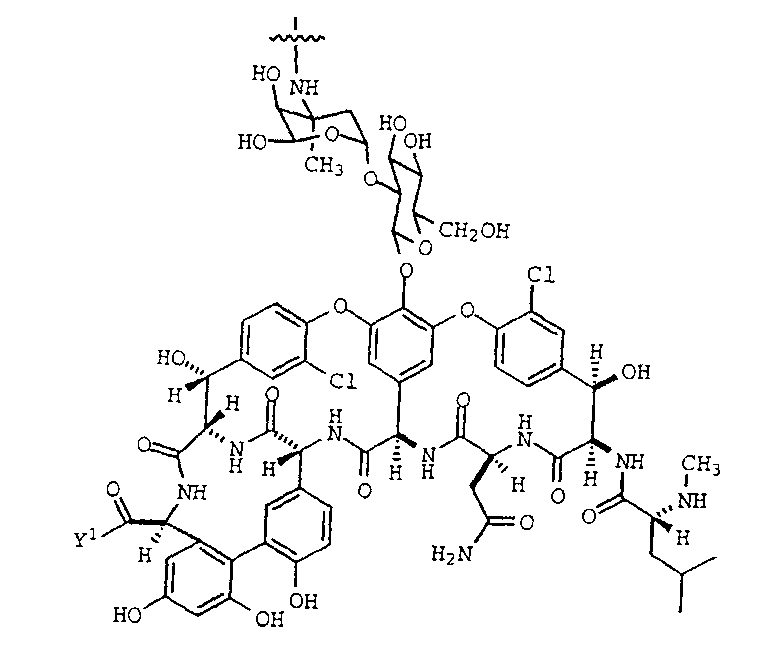 oder Deshydro A82846B der Formel 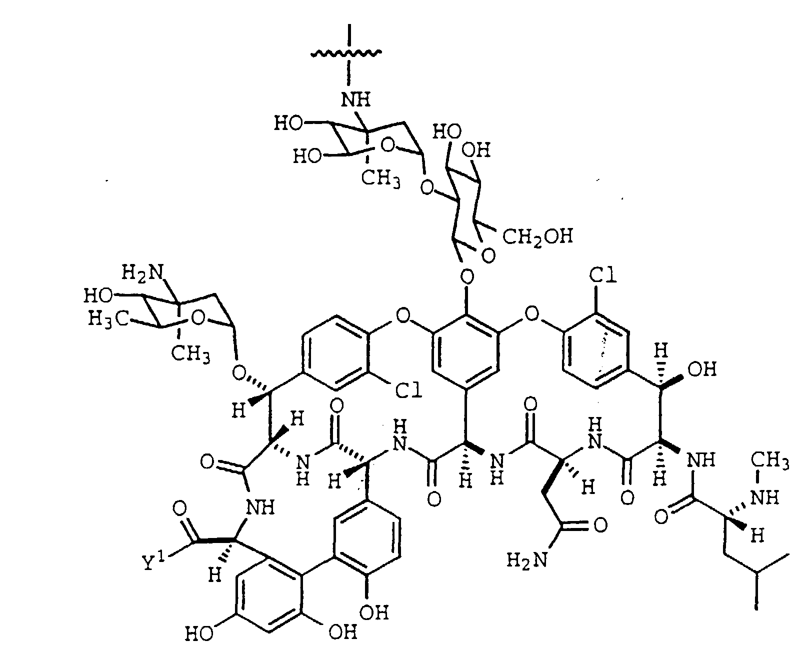 worin Y¹ für OH oder 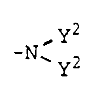 steht und Y² wie folgt definiert ist
(1) jedes Y² steht unabhängig für
Wasserstoff,
C₁-C₁₀ Alkyl
C₅-C₆ Cycloalkyl,
C₅-C₆ Cycloalkenyl,
Naphthyl,
Biphenylyl,
einen Rest der Formel -Y³-(Y⁴)_{0,1 oder 2,} worin Y³ für ein C₁-C₆ Niederalkyl steht, das wahlweise mit einem bis drei Substituenten substituiert ist, wobei jeder unabhängig aus der Gruppe ausgewählt ist, die besteht aus Halogen, Nitro, Cyano, Alkoxy, Halogenalkyl und Halogenalkoxy, und Y¹ für 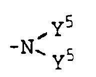 steht, worin Y⁵ unabhängig für Wasserstoff oder C₁-C₄ Niederalkyl steht, oder Y⁴ für Phenyl oder Phenyl steht, das mit einem bis drei Substituenten substituiert ist, die jeweils unabhängig sind
Halogen,
Nitro,
C₁-C₄ Niederalkyl,
C₅-C₆Cycloalkyl,
C₁-C₄ Niederalkoxy,
C₁-C₄ Halogenniederalkyl, oder
C₁-C₄ Halogenniederalkoxy, oder
(2) ein Y² für Wasserstoff steht und das andere Y² für (2-Furanon-3-yl) steht, oder
(3) beide Y² werden mit dem Stickstoff zusammengenommen und bilden einen fünf- bis siebengliedrigen heterocyclischen Ring, der wahlweise zusätzlich zum angegebenen Stickstoffatom ein weiteres Heteroringatom enthält, das Stickstoff, Sauerstoff oder Schwefel ist und worin der heterocyclische Rest unsubstituiert oder substituiert ist mit einem oder zwei Substituenten, wobei jeder hiervon ein C₁-C₂ Niederalkyl, ein C₁-C₂ Niederalkoxy, Phenyl, Benzyl oder C₁-C₆ Alkanoyl ist und L für einen divalenten Brückenrest der Formel A steht: 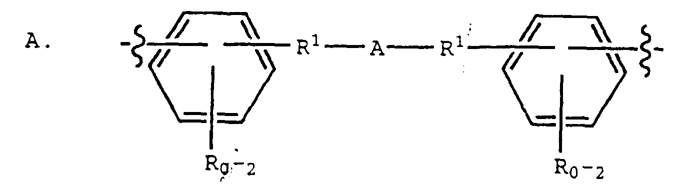
worin A steht für
C₁-C₁₆ Alkylen,
(C₁-C₄-Alkylen-X')_{q}-C₁-C₄-Alkylen, worin q für 1-3 steht, 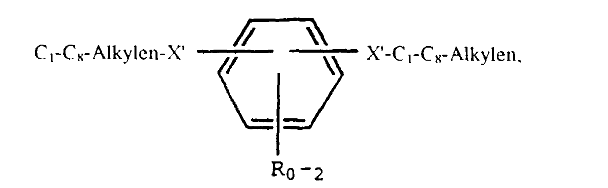 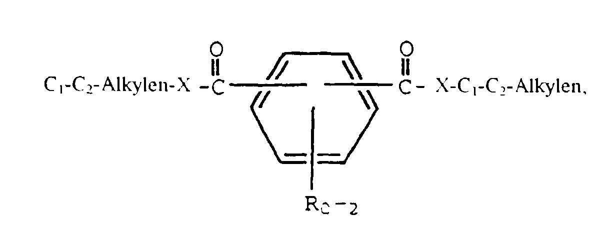 oder 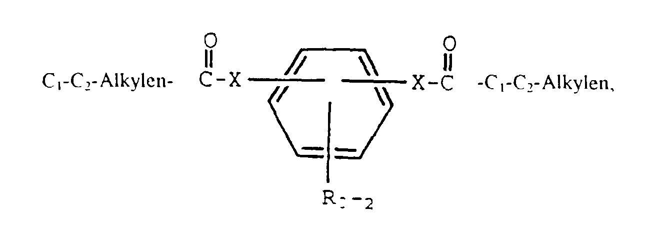 worin jedes R¹ unabhängig steht für CH₂, O, S, 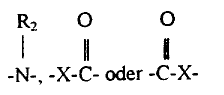 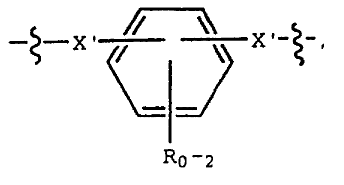 oder 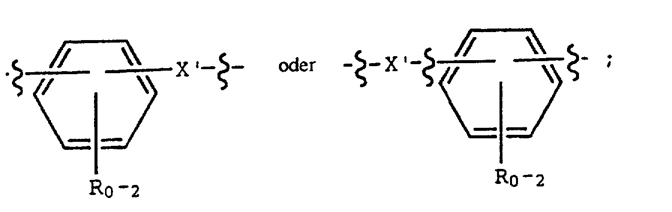 worin jedes R unabhängig für Halogen, C₁-C₆ Niederalkyl, C₁-C₆ Niederalkoxy, Phenyl oder Phenyl steht, das mit 1 bis 2 Substituenten substituiert ist, die jeweils unabhängig Halogen, C₁-C₆ Niederalkyl oder C₁-C₆ Niederalkoxy sind, worin jedes X unabhängig für -O- oder 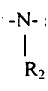 steht, worin
R₂ für H oder C₁-C₄ Niederalkyl steht und jedes X' unabhängig für -O-, -S- oder 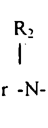 steht, worin R₂ wie oben definiert ist oder L für einen divalenten Brückenrest der Formel B steht:
B. C₁-C₈-Alkylen-R³-X"-R³-C₁-C₈-Alkylen-
worin X" für ein C₁-C₄ Alkylen oder ein Phenylen der folgenden Formel steht 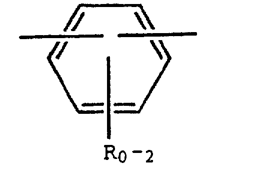 worin R wie oben definiert ist und jedes R³ unabhängig für CH₂ oder O steht oder ein Salz hiervon.

2. Verbindung nach Anspruch 1, worin sowohl G als auch G' für DeshydroA82846B stellen.

3. Verbindung nach Anspruch 1 oder 2, worin L für einen Brückenrest der Formel A steht, A für C₁-C₁₆ Alkylen steht und beide R¹ für O stehen.

4. Verbindung nach Anspruch 3, worin A für ein geradkettiges C₆-C₁₂ Alkylen steht.

5. Verbindung nach Anspruch 1 oder 2, worin L für einen Brückenrest der Formel A steht, A für (C₁-C₄-X' Alkylen)_{q}-C₁-C₄ Alkylen steht, q für 2 steht und beide R¹ für O stehen.

6. Verbindung der Formel
G-CH₂-L-CHO
worin G aus der Gruppe ausgewählt ist, die besteht aus Deshydrovancomycin der Formel 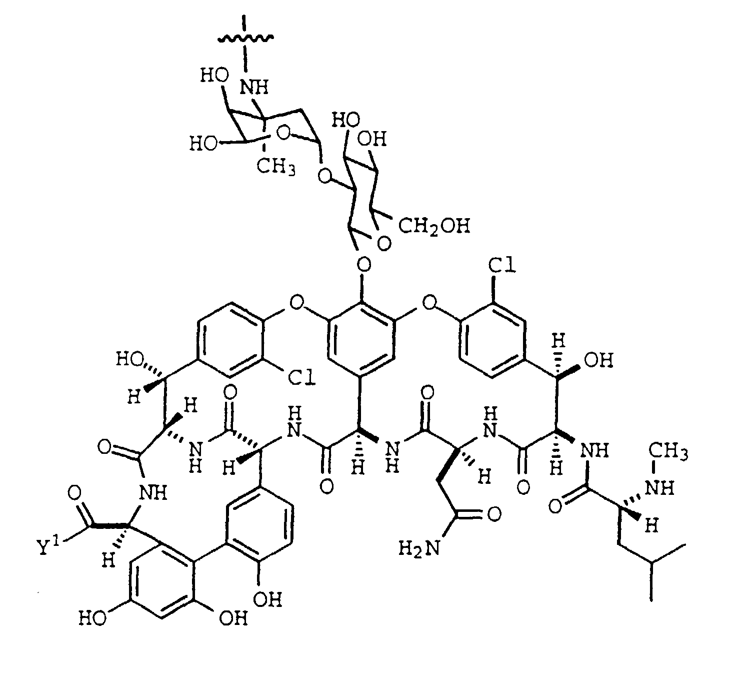 und DeshydroA82846B der Formel 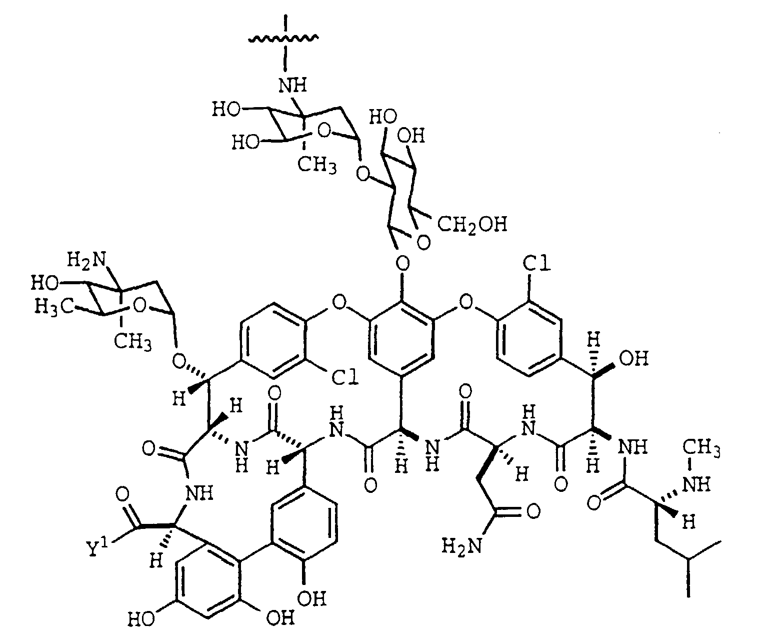 worin Y¹ fur OH oder 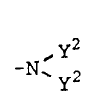 steht und Y² wie folgt definiert ist
(1) jedes Y² steht unabhängig für
Wasserstoff,
C₁-C₁₀, Alkyl
C₅-C₆ Cycloalkyl,
C₅-C₆ Cycloalkenyl,
Naphthyl,
Biphenylyl,
einen Rest der Formel -Y³-(Y⁴)_{0,1 oder 2,} worin Y³ für ein C₁-C₆ Niederalkyl steht, das wahlweise mit einem bis drei Substituenten substituiert ist, wobei jeder unabhängig aus der Gruppe ausgewählt ist, die besteht aus Halogen, Nitro, Cyano, Alkoxy, Halogenalkyl und Halogenalkoxy, und Y⁴ für 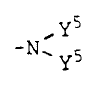 steht, worin Y⁵ unabhängig für Wasserstoff oder C₁-C₄ Niederalkyl steht, oder Y⁴ für Phenyl oder Phenyl steht, das mit einem bis drei Substituenten substituiert ist, die jeweils unabhängig sind
Halogen,
Nitro,
C₁-C₄ Niederalkyl,
C₅-C₆ Cycloalkyl,
C₁-C₄ Niederalkoxy,
C₁-C₄ Halogenniederalkyl, oder
C₁-C₄ Halogenniederalkoxy, oder
(2) ein Y² für Wasserstoff steht und das andere Y² für (2-Furanon-3-yl) steht, oder
(3) beide Y² zusammen mit dem Stickstoff einen fünf- bis siebengliedrigen heterocyclischen Ring bilden, der wahlweise zusätzlich zum angegebenen Stickstoffatom ein weiteres Heteroringatom enthält, das Stickstoff. Sauerstoff oder Schwefel ist und worin der heterocyclische Rest unsubstituiert oder substituiert ist mit einem oder zwei Substituenten, wobei jeder hiervon ein C₁-C₂ Niederalkyl, ein C₁-C₂ Niederalkoxy, Phenyl, Benzyl oder C₁-C₆ Alkanoyl ist und L für einen divalenten Brückenrest der Formel A steht: 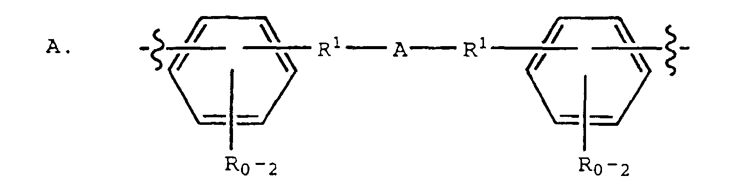
worin A steht für
C₁-C₁₆ Alkylen,
(C₁-C₄-Alkylen-X')_{q}-C₁-C₄-Alkylen, worin q für 1-3 steht, 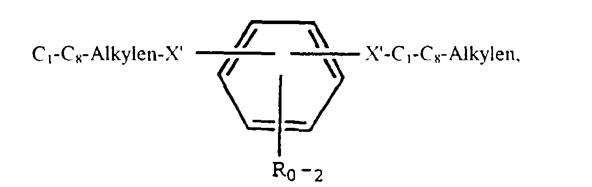 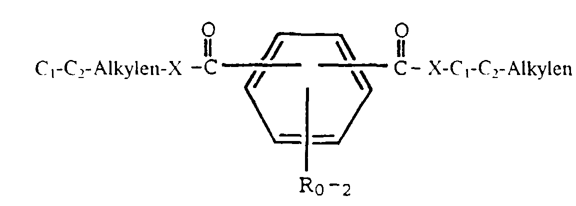 oder 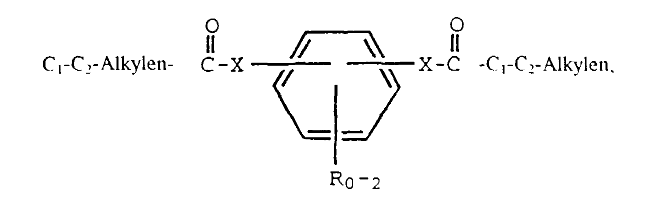 worin jedes R¹ unabhängig steht für CH₂, O, S, 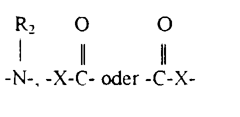 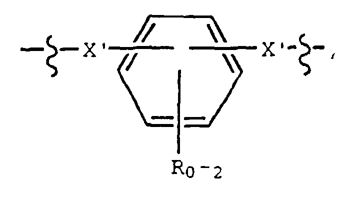 oder 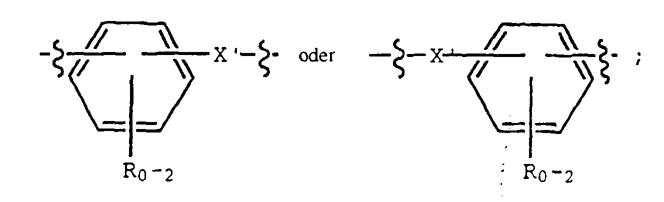 worin jedes R unabhängig für Halogen. C₁-C₆ Niederalkyl, C₁-C₆ Niederalkoxy, Phenyl oder Phenyl steht, das mit 1 bis 2 Substituenten substituiert ist, die jeweils unabhängig Halogen, C₁-C₆ Niederalkyl oder C₁-C₆ Niederalkoxy sind, worin jedes X unabhängig für -O- oder 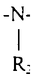 steht, worin
R₂ für H oder C₁-C₄ Niederalkyl steht und jedes X' unabhängig für -O-, -S- oder 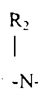 steht, worin R² wie oben definiert ist oder L für einen divalenten Brückenrest der Formel B steht:
B. C₁-C₈-Alkylen-R³-X"-R³-C₁-C₈-Alkylen-
worin X" für ein C₁-C₄ Alkylen oder ein Phenylen der folgenden Formel steht 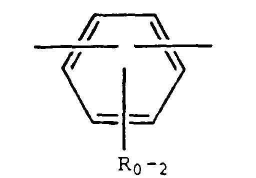 worin R wie oben definiert ist und jedes R³ unabhängig für CH₂ oder O steht oder ein Salz hiervon.

7. Pharmazeutische Formulierung, die eine Verbindung nach Anspruch 1 bis 6 in Kombination mit einem pharmazeutisch annehmbaren Verdünnungsmittel oder Träger enthält.

8. Verbindung nach einem der Ansprüche 1 bis 6 zur Verwendung als Pharmazeutikum.

9. Verbindung nach einem der Ansprüche 1 bis 6 zur Verwendung bei der Behandlung einer bakteriellen Infektion in einem Wirt.

10. Verbindung zur Verwendung nach Anspruch 9, worin die bakterielle Infektion einem Vancomycin-resistenten Enterococcus zuzuschreiben ist

11. Verbindung nach Anspruch 1 oder 6 zur Verwendung in einer antibakteriellen Therapie.

12. Verbindung nach Anspruch 1 oder 6 zur Verwendung in einer antibakteriellen Therapie gegen einen Vancomycin-resistenten Enterococcus.

13. Verfahren zur Herstellung einer Verbindung nach einem der Ansprüche 1-6, gekennzeichnet durch die Reduzierung einer Schiffschen Base, die der gewünschten Verbindung nach den Ansprüchen 1-6 entspricht, und falls erwünscht, eine anschließende Bildung eines pharmazeutisch annehmbaren Salzes.

## Revendications

1. Composé répondant à la formule: 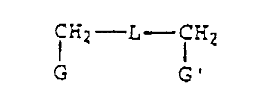 dans laquelle chaque radical G et G' représente, indépendamment l'un de l'autre, la déshydrovancomycine répondant à la formule: 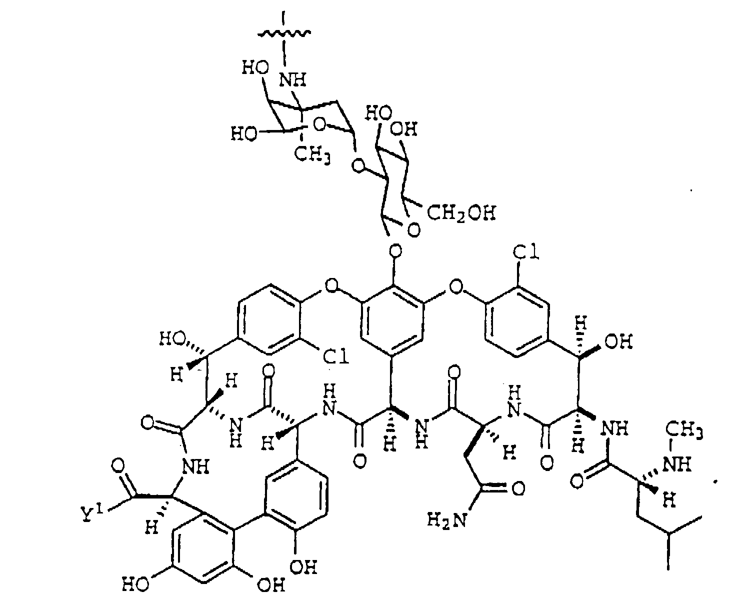 ou le déshydroA82846B répondant à la formule: 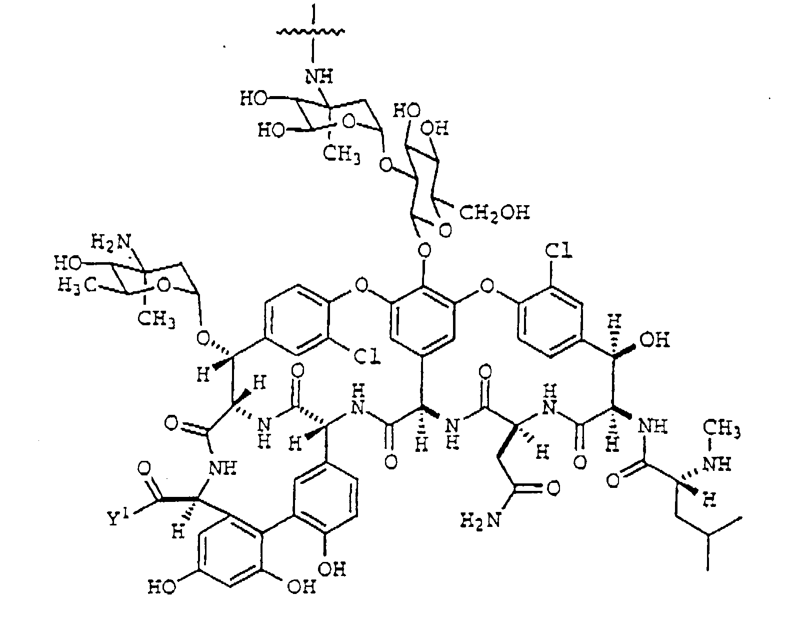 dans lesquelles Y¹ représente un groupe OH ou un groupe 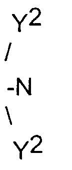 et Y² est tel que défini comme suit:
(1) chaque radical Y² représente, indépendamment l'un de l'autre,
un atome d'hydrogène,
un groupe alkyle en C₁-C₁₀,
un groupe cycloalkyle en C₅-C₆,
un groupe cycloalcényle en C₅-C₆,
un groupe naphtyle,
un groupe biphénylyle,
un radical répondant à la formule -Y³-(Y⁴)_{0, 1 ou 2,} Y³ représentant un groupe alkyle inférieur en C₁-C₆ portant le cas échéant de 1 à 3 substituants identiques ou différents, chacun de ces derniers étant choisi, indépendamment l'un de l'autre, parmi le groupe constitué par un atome d'halogène, par un groupe nitro, par un groupe cyano, par un groupe alcoxy, par un groupe halogénalkyle et par un groupe halogénalcoxy; et Y⁴ représente un groupe 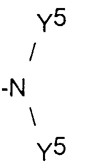 dans lequel chaque radical Y⁵ représente, indépendamment l'un de l'autre, un atome d'hydrogène ou un groupe alkyle inférieur en C₁-C₄, ou bien Y⁴ représente un groupe phényle ou un groupe phényle portant de 1 à 3 substituants identiques ou différents, chacun de ces derniers étant choisi, indépendamment l'un de l'autre, parmi le groupe constitué par
un atome d'halogène,
un groupe nitro,
un groupe alkyle inférieur en C₁-C₄,
un groupe cycloalkyle en C₅-C₆,
un groupe alcoxy inférieur en C₁-C₄,
un groupe halogénalkyle inférieur en C₁-C₄ ou par
un groupe halogénoalcoxy inférieur en C₁-C₄; ou bien
(2) un radical Y² représente un atome d'hydrogène, l'autre radical Y² représentant un groupe (2-furanon-3-yle); ou bien
(3) les deux radicaux Y² sont pris ensemble avec l'atome d'azote et constituent un noyau hétérocyclique de 5 à 7 membres contenant le cas échéant, en plus de l'atome d'azote indiqué, un hétéroatome cyclique supplémentaire qui représente un atome d'azote, un atome d'oxygène ou un atome de soufre, ledit radical hétérocyclique pouvant être non substitué ou porter un ou deux substituants identiques ou différents représentant respectivement un groupe alkyle inférieur en C₁-C₂, un groupe alcoxy inférieur en C₁-C₂, un groupe phényle, un groupe benzyle ou un groupe alcanoyle en C₁-C₆; et L représente un radical de liaison divalent répondant à la formule A: 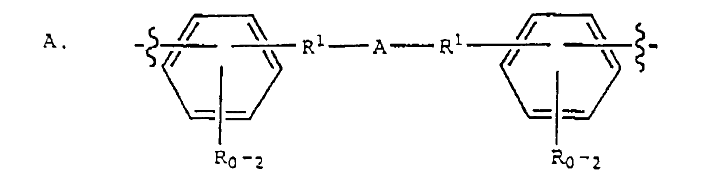
dans laquelle A représente:
un groupe alkyléne en C₁-C₁₆,
un groupe (alkylène en C₁-C₄-X')_{q}-alkylène en C₁-C₄ où q représente 1-3,
un groupe alkylène en 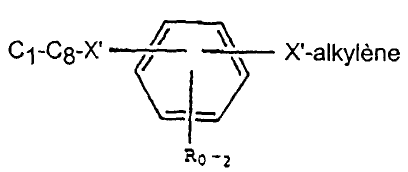 en C₁-C₈
un groupe alkyléne en 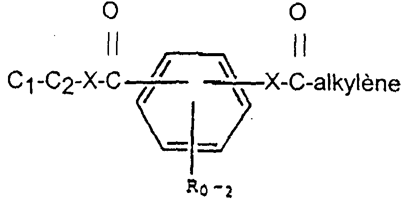 en C₁-C₂ ou
un groupe alkyléne en 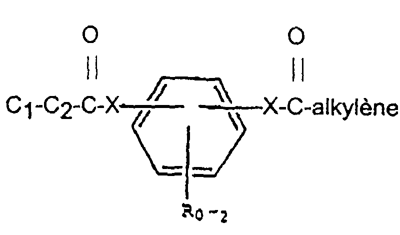 en C₁-C₂
chaque radical R¹ représente de manière indépendante
un groupe CH₂,
un atome d'oxygène,
un atome de soufre,
un groupe 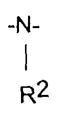
un groupe 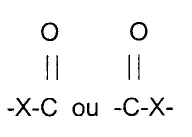 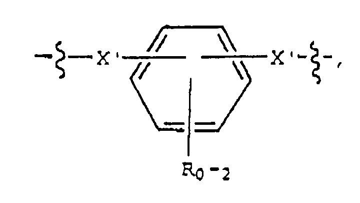 ou 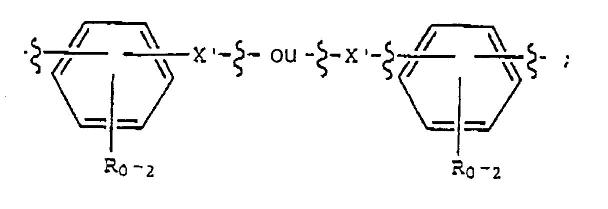
dans lesquels chaque radical R représente, indépendamment l'un de l'autre, un atome d'halogène, un groupe alkyle inférieur en C₁-C₆, un groupe alcoxy inférieur en C₁-C₆, un groupe phényle ou un groupe phényle portant de 1 à 2 substituants identiques ou différents, chacun de ces derniers étant choisi, indépendamment l'un de l'autre, parmi le groupe constitué par un atome d'halogène, par un groupe alkyle inférieur en C₁-C₆ ou par un groupe alcoxy inférieur en C₁-C₆; chaque radical X représente, indépendamment l'un de l'autre, un atome d'oxygène ou un groupe 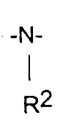
où R² représente un atome d'hydrogène ou un groupe alkyle inférieur en C₁-C₄; et chaque radical X' représente, indépendamment l'un de l'autre, un atome d'oxygène, un atome de soufre ou un groupe 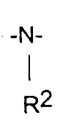 où R² est tel que défini ci-dessus; ou L représente un radical de liaison divalent répondant à la formule B:
B. -alkylène en C₁-C₈-R³-X"-R³-alkylène en C₁-C₈-
où X" représente un groupe alkyléne en C₁-C₄ ou un groupe phénylène répondant à la formule: 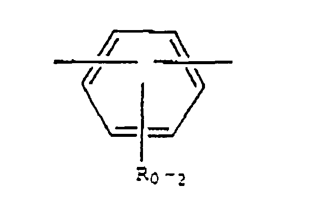 dans laquelle R est tel que défini ci-dessus et chaque radical R³ représente, indépendamment l'un de l'autre, un groupe CH₂ ou un atome d'oxygène, ou un de ses sels.

2. Composé selon la revendication 1, dans lequel les deux radicaux G et G' représentent le déshydroA82846B.

3. Composé selon la revendication 1 ou 2, dans lequel L représente un radical de liaison répondant à la formule A; A représente un groupe alkylène en C₁-C₁₆ et les deux radicaux R¹ représentent un atome d'oxygène.

4. Composé selon la revendication 3, dans lequel A représente un groupe alkylène en C₆-C₁₂ à chaîne droite.

5. Composé selon la revendication 1 ou 2, dans lequel L représente un radical de liaison répondant à la formule A, A représente un groupe (alkyléne en C₁-C₄-X')_{q}-alkylène en C₁-C₄, q = 2 et les deux radicaux R¹ représentent un atome d'oxygène.

6. Composé répondant à la formule:
G-CH₂-L-CHO
dans laquelle G est choisi parmi le groupe constitué par la déshydrovancomycine répondant à la formule: 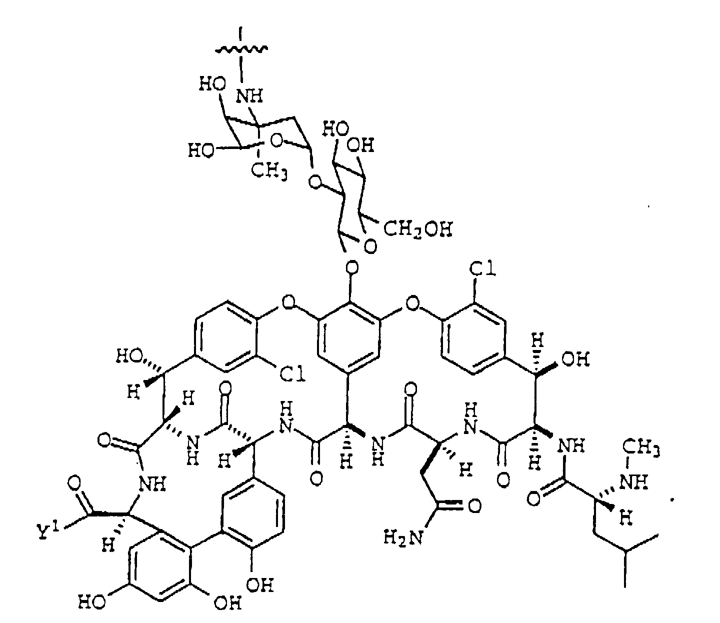 et par le déshydroA82846B répondant à la formule: 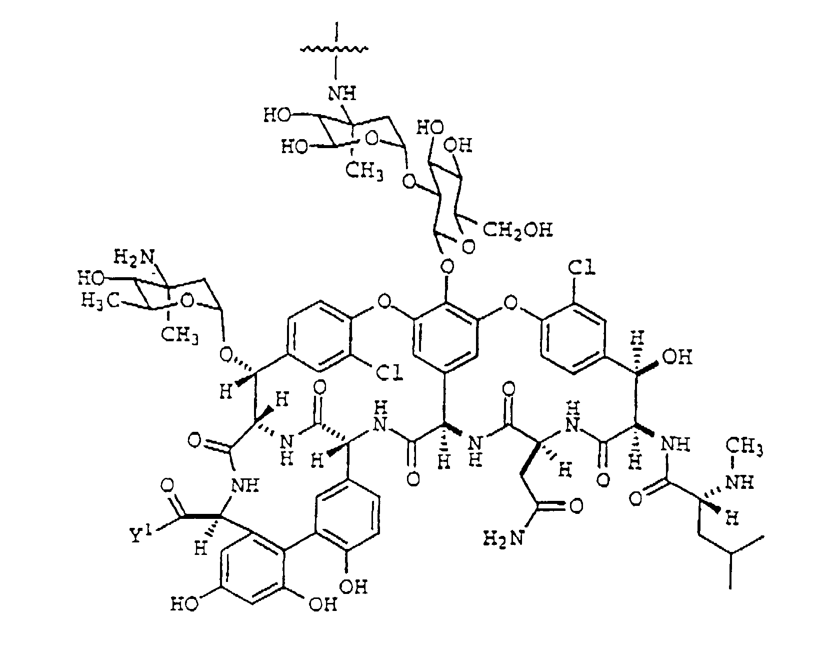
dans laquelle Y¹ représente un groupe OH ou un groupe 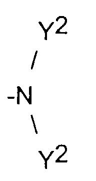
et Y² est tel que défini comme suit:
(1) chaque radical Y² représente, indépendamment l'un de l'autre,
un atome d'hydrogène,
un groupe alkyle en C₁-C₁₀,
un groupe cycloalkyle en C₅-C₆,
un groupe cycloalcényle en C₅-C₆,
un groupe naphtyle,
un groupe biphénylyle,
un radical répondant à la formule -Y³-(Y⁴)_{0, 1 ou 2,} Y³ représentant un groupe alkyle inférieur en C₁-C₆ portant le cas échéant de 1 à 3 substituants identiques ou différents, chacun de ces derniers étant choisi, indépendamment l'un de l'autre, parmi le groupe constitué par un atome d'halogène, par un groupe nitro, par un groupe cyano, par un groupe alcoxy, par un groupe halogénalkyle et par un groupe halogénalcoxy; et Y⁴ représente un groupe 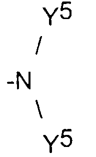 dans lequel chaque radical Y⁵ représente, indépendamment l'un de l'autre, un atome d'hydrogène ou un groupe alkyle inférieur en C₁-C₄, ou bien Y⁴ représente un groupe phényle ou un groupe phényle portant de 1 à 3 substituants identiques ou différents, chacun de ces derniers étant choisi, indépendamment l'un de l'autre, parmi le groupe constitué par
un atome d'halogène,
un groupe nitro,
un groupe alkyle inférieur en C₁-C₄,
un groupe cycloalkyle en C₅-C₆,
un groupe alcoxy inférieur en C₁-C₄,
un groupe halogénalkyle inférieur en C₁-C₄ ou par
un groupe halogénoalcoxy inférieur en C₁-C₄; ou bien
(2) un radical Y² représente un atome d'hydrogène, l'autre radical Y² représentant un groupe (2-furanon-3-yle); ou bien
(3) les deux radicaux Y² sont pris ensemble avec l'atome d'azote et constituent un noyau hétérocyclique de 5 à 7 membres contenant le cas échéant, en plus de l'atome d'azote indiqué, un hétéroatome cyclique supplémentaire qui représente un atome d'azote, un atome d'oxygène ou un atome de soufre, ledit radical hétérocyclique pouvant être non substitué ou porter un ou deux substituants identiques ou différents représentant respectivement un groupe alkyle inférieur en C₁-C₂, un groupe alcoxy inférieur en C₁-C₂, un groupe phényle, un groupe benzyle ou un groupe alcanoyle en C₁-C₆; et L représente un radical de liaison divalent répondant à la formule A: 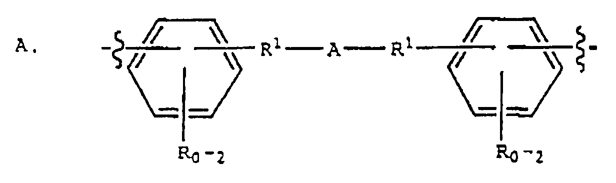
dans laquelle A représente:
un groupe alkylène en C₁-C₁₆,
un groupe (alkylène en C₁-C₄-X')_{q}-alkylène en C₁-C₄ où q représente 1-3,
un groupe alkyléne en 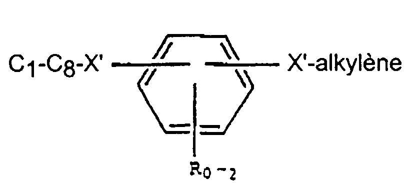 en C₁-C₈
un groupe alkylène en 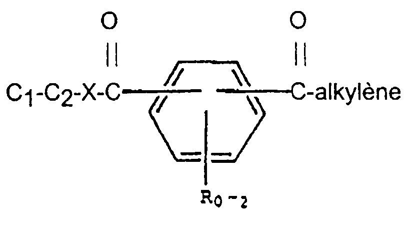 en C₁-C₂ ou
un groupe alkylène en 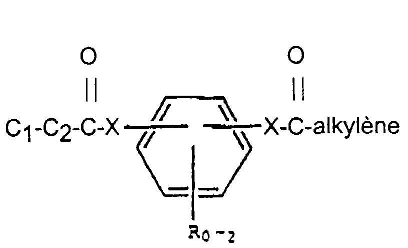
en C₁-C₂ chaque radical R¹ représente de manière indépendante
un groupe CH₂,
un atome d'oxygène,
un atome de soufre,
un groupe 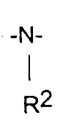
un groupe 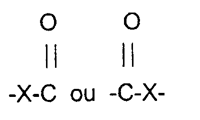 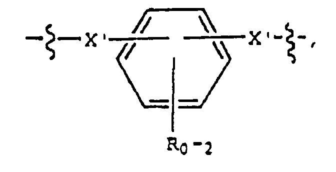 ou 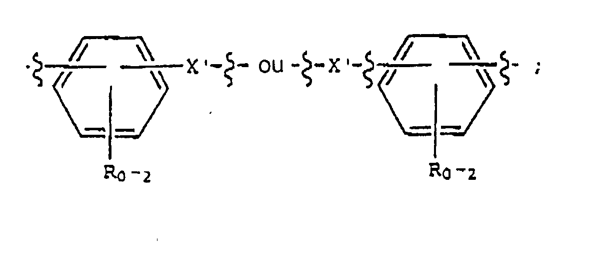 dans lesquels chaque radical R représente, indépendamment l'un de l'autre, un atome d'halogène, un groupe alkyle inférieur en C₁-C₆, un groupe alcoxy inférieur en C₁-C₆, un groupe phényle ou un groupe phényle portant de 1 à 2 substituants identiques ou différents, chacun de ces derniers étant choisi, indépendamment l'un de l'autre, parmi le groupe constitué par un atome d'halogène, par un groupe alkyle inférieur en C₁-C₆ ou par un groupe alcoxy inférieur en C₁-C₆; chaque radical X représente, indépendamment l'un de l'autre, un atome d'oxygène ou un groupe 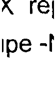 où R² représente un atome d'hydrogène ou un groupe alkyle inférieur en C₁-C₄; et chaque radical X' représente, indépendamment l'un de l'autre, un atome d'oxygène, un atome de soufre ou un groupe 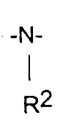 où R² est tel que défini ci-dessus; ou L représente un radical de liaison divalent répondant à la formule B:
B. -alkylène en C₁-C₈-R³-X"-R³-alkylène en C₁-C₈-
où X" représente un groupe alkyléne en C₁-C₄ ou un groupe phénylène répondant à la formule: 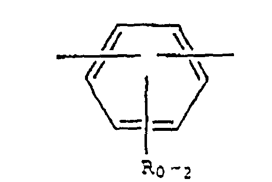 dans laquelle R est tel que défini ci-dessus et chaque radical R³ représente, indépendamment l'un de l'autre, un groupe CH₂ ou un atome d'oxygène, ou un de ses sels.

7. Formulation pharmaceutique comprenant un composé selon la revendication 1 ou 6 en combinaison avec un diluant ou un support pharmaceutiquement acceptable.

8. Composé selon l'une quelconque des revendications 1 à 6 à utiliser comme produit pharmaceutique.

9. Composé selon l'une quelconque des revendications 1 à 6 à utiliser dans le traitement d'une infection bactérienne chez un hôte.

10. Composé à utiliser conformément à la revendication 9, dans lequel l'infection bactérienne peut être attribuée à un entérocoque résistant à la vancomycine.

11. Composé selon la revendication 1 ou 6 à utiliser dans une thérapie antibactérienne.

12. Composé selon la revendication 1 ou 6 à utiliser dans une thérapie antibactérienne contre un entérocoque résistant à la vancomycine.

13. Procédé pour la préparation d'un composé selon l'une quelconque des revendications 1 à 6, qui comprend le fait de réduire une base de Schiff correspondant au composé désiré selon les revendications 1 à 6 et, si on le souhaite, former par la suite un sel pharmaceutiquement acceptable.
